(19) **Europäisches Patentamt**
**European Patent Office**
**Office européen des brevets**

(11) **EP 2 180 865 B1**

(12) **EUROPÄISCHE PATENTSCHRIFT**

(45) Veröffentlichungstag und Bekanntmachung des Hinweises auf die Patenterteilung:
**08.02.2012 Patentblatt 2012/06**

(21) Anmeldenummer: **08865330.8**

(22) Anmeldetag: **04.12.2008**

(51) Int Cl.:
***A61F 13/496*** (2006.01)

(86) Internationale Anmeldenummer:
**PCT/EP2008/010256**

(87) Internationale Veröffentlichungsnummer:
**WO 2009/080180 (02.07.2009 Gazette 2009/27)**

(54) **VERFAHREN ZUM HERSTELLEN VON INKONTINENZARTIKELN IN HÖSCHENFORM**

METHOD FOR PRODUCING INCONTINENCE PANTS

PROCÉDÉ DE FABRICATION D'ARTICLES POUR INCONTINENTS SOUS FORME DE SLIPS

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **20.12.2007 DE 102007063209**

(43) Veröffentlichungstag der Anmeldung:
**05.05.2010 Patentblatt 2010/18**

(73) Patentinhaber: **Paul Hartmann Aktiengesellschaft 89522 Heidenheim (DE)**

(72) Erfinder:
• **HORNUNG, Fridmann Santiagot (CL)**

• **WENZEL, Benjamin 89231 Neu-Ulm (DE)**
• **OSTERTAG, Wolfgang 89547 Gerstetten (DE)**

(74) Vertreter: **Friz, Oliver Dreiss Patentanwälte Postfach 10 37 62 70032 Stuttgart (DE)**

(56) Entgegenhaltungen:
**JP-A- 2003 126 148    US-A1- 2002 151 864 US-A1- 2006 254 708**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein kontinuierliches Verfahren zum Herstellen von Inkontinenzartikeln in Höschenform für die Aufnahme von Körperausscheidungen, mit einem vorderen Bauchabschnitt und einem hinteren Rückenabschnitt, die zur Bildung eines in Quer- oder Hüftumfangsrichtung durchgehenden Bauch- und Rükkenbands mit einer in Hüftumfangsrichtung geschlossenen Hüftöffnung an beidseitigen Seitennahtbereichen herstellerseitig miteinander verbunden sind, und mit einem einen Absorptionskörper aufweisenden Schrittabschnitt, der in einer Längsrichtung zwischen Bauchabschnitt und Rückenabschnitt erstreckt und an den Bauchabschnitt und an den Rückenabschnitt unlösbar angefügt ist, wobei sowohl der Schrittabschnitt als auch der Bauchabschnitt und der Rückenabschnitt die Beinöffnungen des Inkontinenzartikels begrenzen, wobei in dem Bauchabschnitt und dem Rückenabschnitt erste Elastifizierungsmittel vorgesehen sind, die sich in einem Abstand voneinander und parallel zueinander in Quer- oder Hüftumfangsrichtung erstrecken und so den Bauchbereich und den Rückenbereich flächenhaft elastifizieren, wobei in einem schrittseitigen und den Beinöffnungen zugewandten Bereich des Bauchabschnitts und des Rückenabschnitts (also hüftfern) zweite Elastifizierungsmittel vorgesehen sind, die sich ausgehend von den beiden Seitennahtbereichen in Richtung auf eine Längsmittelachse des Inkontinenzartikels erstrecken und dabei bogenförmig sich auffächernd mit zunehmendem Abstand voneinander verlaufen.

Ein derartiger dreikomponentiger Inkontinenzartikel ist beispielsweise in der nicht vorveröffentlichten deutschen Patentanmeldung DE 10 2007 055524.7 der Anmelderin beschrieben.

Bei diesem spezifischen Produktkonzept kann nach dem Anfügen des in Längsrichtung erstreckten Schrittabschnitts an den im Wesentlichen in Quer- oder Hüftumfangsrichtung erstreckten Bauchabschnitt und den entsprechend erstreckten Rückenabschnitt im eben ausgebreiteten Zustand dieser drei Komponenten eine H-förmige Grundstruktur des Inkontinenzartikels realisiert werden. Der Inkontinenzartikel ist dann modular aus den Komponenten Schrittabschnitt, Bauchabschnitt und Rückenabschnitt gebildet. Diese Komponenten werden vorteilhafterweise zunächst über den Schrittabschnitt miteinander verbunden, und vorzugsweise danach wird in beidseitigen Seitennahtbereichen der Bauchabschnitt mit dem Rückenabschnitt verbunden. Hierbei handelt es sich um eine herstellerseitige Verbindung, durch welche die Höschenform erhalten wird. Diese Verbindung ist typischerweise unlösbar. Die Höschenform kann aber auch insbesondere entlang einer Sollbruchlinie, die insbesondere im Seitennahtbereich verlaufen kann, auftrennbar sein, beispielsweise zum Abnehmen eines gebrauchten Inkontinenzartikels bei einer pflegebedürftigen Person.

Inkontinenzartikel in Höschenform unterscheiden sich prinzipiell von öffenbaren und schließbaren Inkontinenzartikeln in üblicher Windelform dadurch, dass in der Regel der Hüftumfang vorgegeben ist und die Anpassung an unterschiedliche Körpergrößen ausgehend von einer Anzahl von Grundgrößen durch eine elastische Dehnbarkeit des Artikels erreicht wird. Hierfür werden in der Regel Elastifizierungsmittel, insbesondere in Form von Bändern oder Fäden, häufig als Lycra-Fäden bezeichnet, in vorgedehntem Zustand (Stretch-Bond-Verfahren) mit Chassismaterialien des Inkontinenzartikels verbunden, das heißt, sie werden in vorgedehntem Zustand an den Chassismaterialien z.B. mittels Kleber fixiert. Infolge ihrer Vorspannung raffen diese Elastifizierungsmittel die Chassismaterialien zusammen und bilden dabei Fältelungen. Der Inkontinenzartikel bzw. die elastifizierten Chassismaterialien des Inkontinenzartikels lassen sich dann wieder elastisch dehnen, wenn der Inkontinenzartikel wie ein Höschen an den Benutzer angelegt wird. Inkontinenzartikel in Höschenform mit derart elastifizierten Chassismaterialien sind mehrfach bekannt geworden und beispielsweise auch in der WO 2004/052260 A1 behandelt.

Dadurch, dass sowohl der Schrittabschnitt als auch der Bauchabschnitt und der Rückenabschnitt des Inkontinenzartikels die Beinöffnungen begrenzen, erweist sich eine optimale Konturierung der Beinöffnungen herstellungstechnisch als schwierig, insbesondere wenn - wie bei der Herstellung moderner Inkontinenzartikel üblich - mit hohen Maschinengeschwindigkeiten, insbesondere in der Größenordnung von mehreren 100 m/min gearbeitet wird.

Der vorliegenden Erfindung liegt die Aufgabe zugrunde, ein auf wirtschaftliche Weise durchführbares kontinuierliches Verfahren zum Herstellen eines Inkontinenzartikels in Höschenform mit den eingangs genannten Merkmalen anzugeben, welches insbesondere den vorstehend genannten Aspekten der Konturierung der Beinöffnungen Rechnung trägt.

Diese Aufgabe wird erfindungsgemäß gelöst durch ein Verfahren mit den folgenden Verfahrensschritten:

- Zuführen von Absorptionskörpern aufweisenden Schrittabschnitten, welche durch einen ersten Konturschnitt gebildete Beinausschnitte aufweisen,

- Zuführen zweier den späteren Bauchabschnitt bzw. den späteren Rückenabschnitt des Inkontinenzartikels bildenden Teilbahnen auf Vliesbasis,

- Zuführen und Aufbringen der zweiten Elastifizierungsmittel auf die beiden Teilbahnen und Fixieren daran,

- Zusammenführen der Schrittabschnitte mit den beiden Teilbahnen, derart, dass die Schrittabschnitte in einer Längsrichtung quer zur Maschinenrichtung einenends mit der einen Teilbahn und anderenends mit der anderen Teilbahn überlappen und die Schrittabschnitte mit einem Abstand in der Maschinenrichtung voneinander angeordnet sind, und Fixieren der Schrittabschnitte und Teilbahnen in den Überlappungsbereichen, und Weiterfördern in der Maschinenrichtung,

- Zuführen, Aufbringen und Fixieren der ersten Elastifizierungsmittel in der Maschinenrichtung an den Teilbahnen,

- Ausführen eines die Teilbahnen an ihren einander zugewandten Randabschnitten erfassenden zweiten Konturschnitts zur Bildung von im Wesentlichen bogenförmigen Beinausschnitten,

- Falten um eine in Maschinenrichtung verlaufende Faltlinie, derart, dass die eine Teilbahn über die andere Teilbahn zu liegen kommt,

- Fügen der übereinander liegenden Teilbahnen quer zur Maschinenrichtung in Abständen voneinander zur Ausbildung von Seitennahtbereichen der herzustellenden Inkontinenzartikel, und Erhalt von einen Bauchabschnitt, einen Rückenabschnitt und einen dazwischen angeordneten Schrittabschnitt aufweisenden Produkten,

- Ausführen eines Trennschnitts quer zur Maschinenrichtung und Erhalt von vereinzelten fertigen Inkontinenzartikeln.

Dadurch, dass die Ausbildung der Beinausschnitte zweistufig realisiert wird, nämlich durch einen ersten Konturschnitt, der an dem Schrittabschnitt des Hygieneartikels ausgeführt wird, und durch einen zweiten Konturschnitt, der an den beiden Teilbahnen, welche den Bauchabschnitt bzw. den Rückenabschnitt des Inkontinenzartikels bilden, ausgeführt wird, kann die Formgebung optimiert werden. Auf diese Weise ist der jeweilige einzelne Konturschnitt auch kürzer. Außerdem ist es möglich, den einzelnen Schnitt besser an die Maschinenrichtung anzupassen. Zudem ist es möglich, dass die Schneidvorrichtungen für den Konturschnitt in Bezug auf die zu schneidenden Materialien optimiert werden können, z.B. können verschiedene Schneidemesser für verschiedene Materialien eingesetzt werden.
Nach einer bevorzugten Ausführungsform des erfindungsgemäßen Verfahrens wird nach dem Zuführen und Aufbringen der zweiten Elastifizierungsmittel auf die Teilbahnen und Fixieren daran eine die zweiten Elastifizierungsmittel überfangende Vliesdeckschicht auf jede Teilbahn zugeführt und aufgebracht, und die zwei Teilbahnen werden in der Maschinenrichtung in einem Abstand voneinander und parallel zueinander weitergefördert.
Nach einer weiter bevorzugten Ausführungsform des erfindungsgemäßen Verfahrens wird nach dem Zuführen, Aufbringen und Fixieren der ersten Elastifizierungsmittel in der Maschinenrichtung an die Teilbahnen eine die ersten Elastifizierungsmittel überfangende Vliesdeckschicht auf jede Teilbahn aufgebracht und die zwei Teilbahnen werden in der Maschinenrichtung in einem Abstand voneinander und parallel zueinander weitergefördert.
Es wäre grundsätzlich denkbar, dass die zuzu-führenden Schrittabschnitte des Inkontinenzartikels der-art vorkonfiguriert sind, dass sie endlos, insbesondere von einer Rolle, zugeführt werden und bereits den ersten Konturschnitt zur Bildung der Beinausschnitte an dem Schrittabschnitt aufweisen. Es kann sich aber nach einer weiteren Ausführungsform des erfindungsgemäßen Verfahrens als vorteilhaft erweisen, wenn die zuzuführenden Schrittabschnitte insbesondere gleichförmige Breite aufweisen und dann der erste Konturschnitt zur Bildung der Beinausschnitte bei dem Schrittabschnitt innerhalb des kontinuierlichen Verfahrens ausgeführt wird.
Nach einer weiteren Verfahrensvariante kann es sich als vorteilhaft erweisen, wenn die Schrittabschnitte erst innerhalb des kontinuierlichen Verfahrens gebildet werden, indem eine endlose Topsheet-Materialbahn, eine endlose Backsheet-Materialbahn und Absorptionskörper in einer Maschinenrichtung zugeführt und die Absorptionskörper voneinander beabstandet zwischen die Topsheet-Materialbahn und die Backsheet-Materialbahn angeordnet werden und der so gebildete Verbund in sich fixiert wird. Dann wird in dem so gebildeten Verbund der erste Konturschnitt ausgeführt, um die Beinausschnitte der späteren Schrittabschnitte des Inkontinenzartikels zu bilden.
Weiter vorteilhafterweise werden zwischen die Hüllmaterialien des Schrittabschnitts, also zwischen Topsheet-Materialbahn und Backsheet-Materialbahn, Beinelastifizierungsmittel zugeführt.
Weiter werden in vorteilhafter Weise die Beinelastifizierungsmittel derart zugeführt, dass sie mit veränderlichem Abstand zum Absorptionskörper verlaufen und an ihren Längsenden einen größeren Abstand zum Absorptionskörper aufweisen als mittig. Weiter vorteil-hafterweise werden die Beinelastifizierungsmittel bogen-förmig zugeführt.
In besonders vorteilhafter Weise werden die Beinelastifizierungsmittel derart zugeführt, dass die Beinelastifizierungsmittel im Wesentlichen den bogenförmigen Beinausschnitten des späteren Schrittabschnitts folgen.
In einer bevorzugten Ausführungsform des Verfahrens wird der erste Konturschnitt derart ausgeführt, dass im Wesentlichen bogenförmige Beinausschnitte der späteren Schrittabschnitte des Inkontinenzartikels gebildet werden.
In einer besonders bevorzugten Ausführungsform wird der erste Konturschnitt mittels eines Schneidwalzenpaars, also eines Rotationsmessers mit einer Ambosswalze, durchgeführt.
Die vorliegende Erfindung erweist sich gerade bei der Herstellung solcher Inkontinenzartikel als besonders vorteilhaft, bei denen der Schrittabschnitt den Beinöffnungen zugeordnete Beinelastifizierungsmittel aufweist. Wenn nämlich solchenfalls die jeweiligen Schrittabschnitte mit Beinelastifizierungsmitteln versehen und dann vereinzelt werden, so üben die Beinelastifizierungsmittel hohe Zugkräfte auf die Materialien des jeweiligen Schrittabschnitts aus und versuchen, den Schrittabschnitt zusammenzuziehen. Dies ist herstellungstechnisch schwer beherrschbar, und es gestaltet sich um so schwieriger, bei vereinzelten Schrittabschnitten dann die Beinöffnungen zu konturieren. Gemäß der vorliegenden Erfindung wird aber derjenige Teil der Beinöffnungen, der von dem Schrittabschnitt begrenzt wird, schon im Zuge der Ausführung des ersten Konturschnitts gefertigt, und zwar zu einem Zeitpunkt, zu dem die Schrittabschnitte noch nicht vereinzelt, also noch endlos gefördert werden. Bei dieser endlosen Förderung können die vorstehend erläuterten Kräfte, die durch die Beinelastifizierungsmittel hervorgerufen werden, besser beherrscht werden, so dass es im Ergebnis möglich ist, mit einem geringeren technischen Aufwand ein besseres Ergebnis hinsichtlich der Genauigkeit bei der Konturierung der Beinöffnungen zu erzielen.

Es ist grundsätzlich denkbar, dass die Komponenten Topsheet-Materialbahn, Backsheet-Materialbahn und Absorptionskörper in der späteren Längsrichtung des Schrittabschnitts bzw. des Hygieneartikels gefördert werden. Ebenso denkbar ist eine Förderung in Querrichtung. Im ersten Fall ist dann eine 90°-Umlenkung im Zuge des endlosen Fertigungsverfahrens erforderlich, da die weitere Fertigung vorzugsweise in der Querrichtung der Inkontinenzartikel erfolgt. In jedem Fall müssen die Schrittabschnitte quer zur Maschinenrichtung getrennt, also vereinzelt werden, und dann im Abstand voneinander weiter gefördert werden, um mit den endlosen Teilbahnen verbunden zu werden.

Die Backsheet-Materialbahn kann in Form einer flüssigkeitsundurchlässigen Folienbahn zugeführt werden, die vor oder während des kontinuierlichen Verfahrens mit einer Vliesbeschichtung versehen wird, was eine textile Anmutung der körperabgewandten Außenseite des Inkontinenzartikels vermitteln kann. Auch die Anbringung einer bereichsweisen verstärkenden Beschichtung der Backsheet-Materialbahn kann sich als vorteilhaft erweisen. Die verstärkende Beschichtung besteht vorzugsweise aus einem Vliesstoff, insbesondere einem Spinnvlies aus Polypropylen, insbesondere mit einem Flächengewicht von 10 - 20 g/m$^2$ , insbesondere von 12 - 17 g/m$^2$.

Die Backsheet-Materialbahn umfasst insbesondere eine Folie, insbesondere eines Flächengewichts von 18 - 40 g/m$^2$. Insbesondere umfasst die Backsheet-Materialbahn eine im Gebrauch flüssigkeitsdichte aber gleichwohl atmungsaktive, also wasserdampfdurchlässige, insbesondere mikroporöse Folie. Die Wasserdampfdurchlässigkeit der Backsheet-Materialbahn beträgt insbesondere wenigstens 300 g/m$^2$/24h , weiter insbesondere wenigstens 1000 g/m$^2$/24h, weiter insbesondere wenigstens 2000 g/m$^2$/24h, weiter insbesondere wenigstens 3000 g/m$^2$/24h, weiter insbesondere wenigstens 4000 g/m$^2$/24h, weiter insbesondere höchstens 6000 g/m$^2$/24h gemessen nach DIN 53 122-1 (Ausgabe: 2001-08).

Der Absorptionskörper umfasst Körperflüssigkeiten absorbierende Materialien wie natürliche oder synthetische Fasern, insbesondere Zellulosefasern, vorzugsweise in Form von Zellstofffluff. Vorzugsweise umfasst der Absorptionskörper außerdem superabsorbierende Materialien (SAP), insbesondere auf Basis oberflächenvernetzter, teilneutralisierter Polyacrylate.

Als Beinelastifizierungsmittel werden vorzugsweise faden- oder bandförmige Elastifizierungsmittel, wie Gummi- oder Polyetherpolyurethan- oder Polyesterpolyurethanfäden, vorzugsweise Elastikfäden wie Lycra®- oder Spandex®-Fäden eingesetzt. Die Beinelastifizierungsmittel haben vorzugsweise eine Stärke von 300 - 1500 dtex, insbesondere von 500 - 1200 dtex, weiter insbesondere von 500 - 900 dtex.

Die Beinelastifizierungsmittel werden vorzugsweise unter einer Vorspannung von 1,5 - 6,0, insbesondere von 2,5 - 4,5 an den Hüllmaterialien des Schrittabschnitts fixiert.

Zum Zuführen der beiden den späteren Bauchabschnitt und späteren Rückenabschnitt des Inkontinenzartikels bildenden Teilbahnen ist es beispielsweise denkbar, dass jede Teilbahn von einer eigenen Rolle abgerollt und dem Herstellverfahren zugeführt wird. Gemäß einer bevorzugten Verfahrensvariante kann aber auch zunächst eine endlose Vliesbahn zugeführt werden, die dann zur Bildung der zwei Teilbahnen entlang der Maschinenrichtung aufgetrennt wird. Auf diese Weise braucht die Maschine nur mit einer Rolle bestückt zu werden.

Die Teilbahnen auf Vliesbasis des späteren Bauchabschnitts und/oder des späteren Rückenabschnitts sind vorzugsweise entnommen aus der Gruppe der Spinnvliese, Meltblownvliese, SM-Vliese, SMS-Vliese, SMMS-Vliese, Kardenvliese oder Through-Air-bonded Kardenvliese. Besonders bevorzugt wird für die Teilbahnen für den späteren Bauchabschnitt und/oder für den späteren Rückenabschnitt ein Spinnvlies eingesetzt. Besonders bevorzugt werden für die Teilbahnen für den späteren Bauchabschnitt und/oder für den späteren Rückenabschnitt Vliesstoffmaterialien mit einem vorteilhaften Flächengewicht von 10 - 30 g/m$^2$, weiter vorzugsweise von 15 - 25 g/m$^2$ eingesetzt. Besonders bevorzugt werden als Teilbahnen für den späteren Bauchabschnitt und den späteren Rückenabschnitt ein Spinnvlies aus Polypropylen, insbesondere mit einem Flächengewicht von 15 - 25 g/m$^2$ eingesetzt.

Vorzugweise werden die ersten und/oder zweiten Elastifizierungsmittel endlos in Maschinenrichtung zu den Teilbahnen zugeführt. Besonders bevorzugt werden die ersten und die zweiten Elastifierungsmittel endlos in Maschenrichtung an die Teilbahnen zugeführt.

Die erwähnten in Hüftumfangsrichtung verlaufenden ersten Elastifizierungsmittel werden vorzugsweise in einem Abstand von 4 bis 10 mm, insbesondere 4 bis 8 mm, insbesondere 4 bis 6 mm voneinander eingebracht.

Die zweiten Elastifizierungsmittel, die sich in Richtung auf die Längsmittelachse des Inkontinenzartikels bogenförmig auffächern, werden entsprechend in einem variierenden Abstand voneinander (Abstand von unmittelbar nebeneinanderliegenden Elastifizierungsmitteln) zwischen 3 mm und 35 mm eingebracht. Zum Einbringen der Elastifizierungsmittel werden vorzugsweise oszillierend antreibbare Führungsorgane für die Elastifizierungsmittel verwendet.

Vorzugsweise werden die zweiten Elastifizierungsmittel derart eingebracht, dass ein minimaler Abstand der zweiten Elastifizierungsmittel voneinander (also der Abstand von unmittelbar nebeneinander liegenden zweiten Elastifizierungsmitteln) in den späteren Seitennahtbereichen 3 bis 8 mm, insbesondere 3 bis 7 mm und weiter insbesondere 3 bis 6 mm beträgt.

Des Weiteren vorzugsweise werden die zweiten Elastifizierungsmittel derart eingebracht, dass ein maximaler Abstand der zweiten Elastifizierungsmittel voneinander (also der Abstand von unmittelbar nebeneinander liegenden Elastifizierungsmitteln) an einem späteren Absorptionskörperrand oder an einem späteren Längsrand des Schrittabschnitts 7 bis 35 mm, insbesondere 10 bis 32 mm und weiter insbesondere 12 bis 30 mm beträgt.

Vorzugsweise unterscheiden sich die Abstände der zweiten Elastifizierungsmittel im späteren Rückenabschnitt von den Abständen der zweiten Elastifizierungsmittel im späteren Bauchabschnitt. Vorzugsweise ist der maximale Abstand der zweiten Elastifizierungsmittel voneinander im späteren Rückenabschnitt größer als der maximale Abstand der zweiten Elastifizierungsmittel im späteren Bauchabschnitt.

Des Weiteren vorteilhaft ist, wenn die zweiten Elastifizierungsmittel einen Auffächerungsgrad F

$$F=(A-B)/B * 100\%$$

von 50 bis 900 %, insbesondere von 100 bis 700 % und weiter insbesondere von 150 bis 550 % haben.

Dabei ist der Auffächerungsgrad F als Verhältnis der Abstandszunahme (A-B) zum minimalen Abstand (B) in Prozent definiert. Dabei sind die Größen A und B definiert als der Abstand des in Längsrichtung des späteren Inkontinenzartikels äußersten zweiten Elastifizierungsmittels von dem in Längsrichtung innersten zweiten Elastifizierungsmittel (also nicht der Abstand von unmittelbar nebeneinander liegenden zweiten Elastifizierungsmitteln), und zwar A als der maximale Abstand, insbesondere am späteren Längsrand des Schrittabschnitts oder am späteren Absorptionskörperrand, und B als der minimale Abstand, insbesondere im späteren Seitennahtbereich.

Es wurde auch erkannt, dass es sich als vorteilhaft erweist, wenn der Auffächerungsgrad F der zweiten Elastifizierungsmittel im späteren Rückenabschnitt größer ist als im späteren Bauchabschnitt.

Es kann sich auch als vorteilhaft erweisen, wenn die zweiten Elastifizierungsmittel in einem Überlappungsbereich mit dem Absorptionskörper hinsichtlich ihrer elastischen Eigenschaften deaktiviert werden. Diese Deaktivierung kann beispielsweise durch Anbringen einer Anzahl von Trennschnitten durch die zweiten Elastifizierungsmittel im Bereich der Überdeckung mit dem Absorptionskörper realisiert werden. Aber auch andere Trennverfahren, wie z. B. Ultraschallschweißen oder Laserschweißen, sind denkbar. Auch die ersten Elastifizierungsmittel können im Bereich einer Überdeckung mit dem Absorptionskörper hinsichtlich ihrer elastischen Eigenschaften deaktiviert werden.

Der Verlauf der zweiten Elastifizierungsmittel und die Ausführung des zweiten Konturschnitts bei den Teilbahnen ist vorteilhafterweise derart, dass der zweite Konturschnitt entlang und im Abstand (D) zu dem in Längsrichtung jeweils innersten der schrittzugewandten zweiten Elastifizierungsmittel ausgeführt wird. Dieser Abstand (D) beträgt vorteilhafterweise 2 bis 40 mm, insbesondere 3 bis 30 mm und weiter insbesondere 4 bis 20 mm.

Vorteilhafterweise werden die ersten und/oder zweiten Elastifizierungsmittel 28, 40, 42 unter einer Vorspannung von 1,5 - 6,0, insbesondere von 2,5 - 5,0 an den Teilbahnen fixiert. Die Vorspannung ist dabei definiert als Faktor des Dehnungsgrads gegenüber dem ungedehnten/relaxierten Zustand des Elastifizierungsmittels.

Als erste und/oder zweite Elastifizierungsmittel werden vorzugsweise faden- oder bandförmige Elastifizierungsmittel, wie Gummi- oder Polyetherpolyurethanoder Polyesterpolyurethanfäden, vorzugsweise Elastikfäden wie Lycra®- oder Spandex®-Fäden eingesetzt. Die ersten und/oder zweiten Elastifizierungsmittel haben vorzugsweise eine Stärke von 300-1500 dtex, insbesondere von 500-900 dtex, weiter insbesondere von 500 - 600 dtex.

Das Fixieren der ersten und/oder zweiten Elastifizierungsmittel auf den Teilbahnen erfolgt vorzugsweise mittels Klebemittel. Die Klebemittel für das Fixieren der ersten und/oder zweiten Elastifizierungsmittel können dabei vorzugsweise direkt auf die Teilbahnen und/oder Vliesabdeckschichten aufgebracht oder direkt auf die Elastifizierungsmittel aufgebracht werden (Fadenbeleimung). Vorzugsweise werden die ersten Elastifizierungsmittel direkt mit Kleber versehen, insbesondere besprüht, und die zweiten Elastifizierungsmittel werden in zuvor flächenhaft auf die Teilbahnen und/oder Vliesabdeckschichten aufgebrachten Kleber gelegt.

Nach einer weiteren bevorzugten Ausführungsform des erfindungsgemäßen Verfahrens erfasst der zweite Konturschnitt nur die Teilbahnen, nicht aber den Schrittabschnitt. Es ist daher möglich, dass entlang der Beinöffnungen ein unstetiger Verlauf im Übergang vom Schrittabschnitt zum Bauchabschnitt bzw. Rückenabschnitt gebildet wird.

Nach einer bevorzugten Ausführungsform des erfindungsgemäßen Verfahrens ist die Ausführung des zweiten Konturschnitts bei den Teilbahnen derart, dass der bogenförmige Beinausschnitt der Teilbahn des späteren Bauchabschnittes einen anderen Verlauf, insbesondere einen anderen Winkel oder Radius, aufweist als der bogenförmige Beinausschnitt der Teilbahn des späteren Rückenabschnitts.

Nach einer bevorzugten Ausführungsform wird der zweite Konturschnitt zur Bildung der im Wesentlichen bogenförmigen Beinausschnitte derart ausgeführt, dass der zweite Konturschnitt bei der Teilbahn des späteren Bauchabschnitts und bei der Teilbahn des späteren Rükkenabschnitts gleichzeitig ausgeführt wird.

In einer besonders bevorzugten Ausführungsform wird der zweite Konturschnitt mittels eines Schneidwalzenpaares, also eines Rotationsmessers mit einer Ambosswalze, durchgeführt.

Des Weiteren kann es sich als vorteilhaft erweisen, wenn das Fügen der übereinander liegenden Teilbahnen zur Ausbildung von Seitennahtbereichen der herzustellenden Inkontinenzartikel und das Ausführen des Trennschnitts in demselben Verfahrensschritt durchgeführt werden.

Weitere Merkmale, Einzelheiten und Vorteile der Erfindung ergeben sich aus den beigefügten Patentansprüchen und aus der zeichnerischen Darstellung und nachfolgenden Beschreibung eines Inkontinenzartikels in Höschenform und des erfindungsgemäßen Herstellungsverfahrens. In der Zeichnung zeigt:

Figur 1     eine Draufsicht auf einen Inkontinenzartikel, wobei ein Bauchabschnitt, ein Rückenabschnitt und ein die beiden verbindender Schrittabschnitt des Inkontinenzartikels noch nicht zur Bildung einer Höschenform verbunden, sondern in flachgelegtem und ausgedehntem Zustand dargestellt sind;

Figur 2     eine ausschnittsweise Darstellung des Inkontinenzartikels nach Figur 1;

Figur 3     eine Draufsicht auf den Schrittabschnitt des Inkontinenzartikels nach Figur 1 wiederum im flachgelegten und ausgedehnten Zustand;

Figur 4     eine Schnittansicht (schematisch) entlang einer Quermittelachse des Schrittabschnitts mit Schnittebene IV-IV in Figur 3;

Figur 5     eine Figur 4 entsprechende Schnittansicht (schematisch) des Schrittabschnitts mit Schnittebene V-V in Figur 3, mit entfalteten und emporstehenden Barrieremitteln;

Figur 6     eine perspektivische Ansicht (schematisch) des an einen Benutzer angelegten Inkontinenzartikels nach Figur 1;

Figur 7     eine Figur 1 entsprechende Draufsicht des Inkontinenzartikels zur Verdeutlichung der Verbindung von Schrittabschnitt und Bauchabschnitt bzw. Rückenabschnitt;

Figuren    8,9 verdeutlichen beispielhaft die Bestimmung von Rückstellkräften im Bauchabschnitt bzw. Rükkenabschnitt des Inkontinenzartikels;

Figur 10    eine schematische Darstellung des erfindungsgemäßen Verfahrens zur Herstellung des Schrittabschnitts;

Figur 11    eine schematische Darstellung der Zuführung und Anfügung von Teilabschnitten an den Schrittabschnitt und die Ausführung eines zweiten Konturschnitts;

Figur 12    eine schematische Darstellung des Faltens auf Höschenform und des Ausbildens von Seitennahtbereichen und anschließender Vereinzelung der Hygieneartikel und

Figur 13    eine schematische Darstellung einer bereichsweisen Beschichtung eines Backsheetmaterials bei der Herstellung des Schrittabschnitts des Hygieneartikels.

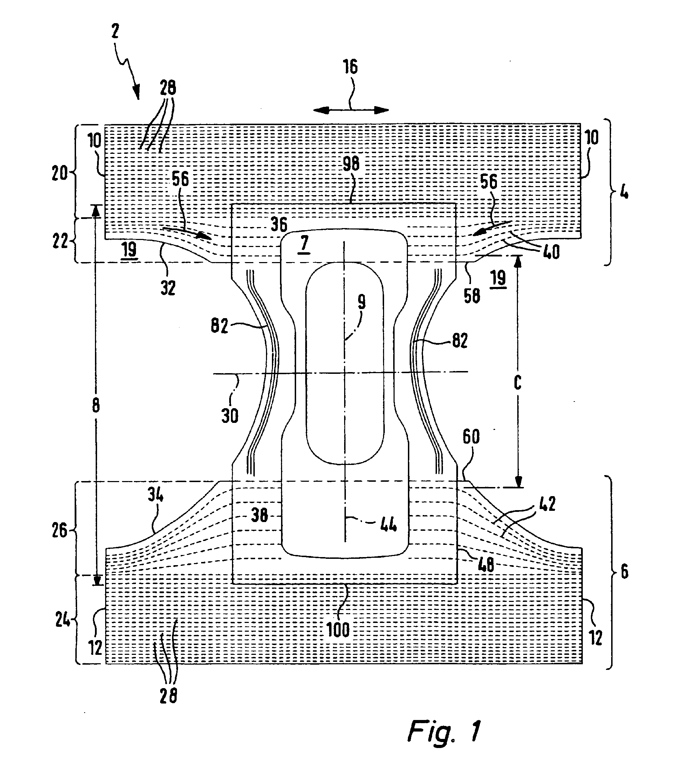

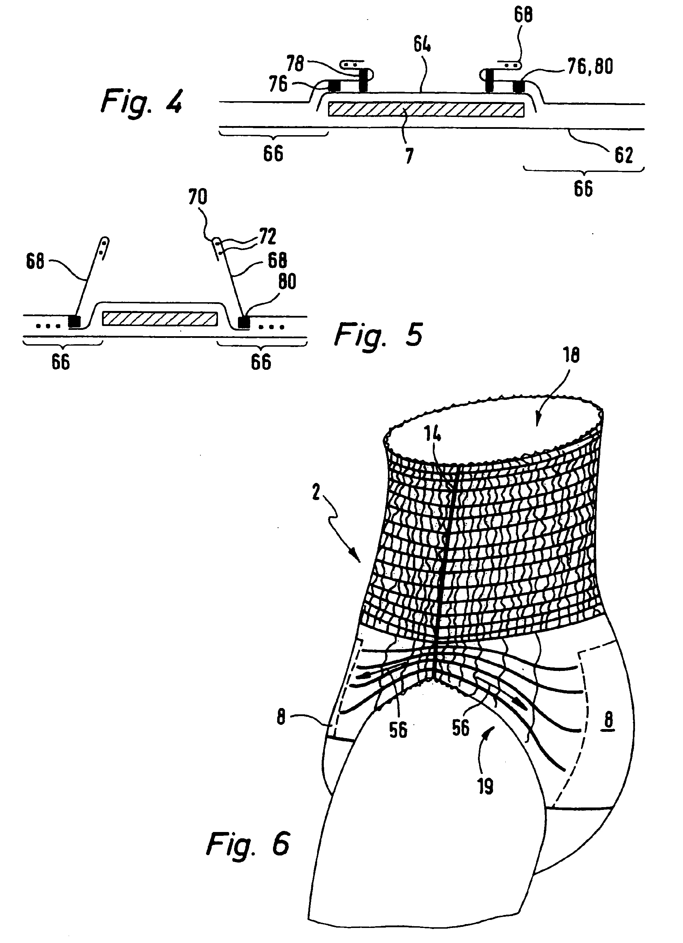

Die Figuren zeigen einen insgesamt mit dem Bezugszeichen 2 bezeichneten Inkontinenzartikel in Höschenform für die Aufnahme fester und flüssiger Körperausscheidungen. Der Inkontinenzartikel 2 ist aus drei weitestgehend unabhängig voneinander fertigbaren Komponenten, nämlich einem vorderen Bauchabschnitt 4, einem hinteren Rückenabschnitt 6 und einem zwischen diesen angeordneten und einen Absorptionskörper 7 aufweisenden Schrittabschnitt 8 gebildet, wobei der Schrittabschnitt 8 mit einem wesentlichen Flächenanteil des Bauchabschnitts 4 einerseits und des Rückenabschnitts 6 andererseits überlappt und im Überlappungsbereich herstellerseitig unlösbar verbunden ist. Wie aus Figur 1 ersichtlich, führt dies zu einer H-förmigen Grundstruktur des Inkontinenzartikels mit einer Längsrichtung 9. Die in Figur 1 dargestellten miteinander gefügten Bestandteile werden dann zur Bildung der in Figur 6 schematisch dargestellten Höschenform an jeweiligen seitlichen Längsrandabschnitten 10, 12 des Bauchabschnitts 4 und des Rückenabschnitts 6 ebenfalls herstellerseitig miteinander verbunden, wodurch beidseits Seitennahtbereiche 14 (Figur 6) ausgebildet werden. In diesem herstellerseitig gefertigten höschenförmigen Zustand des Inkontinenzartikels erstrecken sich der Bauchabschnitt 4 und der Rückenabschnitt 6 in Quer- oder Hüftumfangsrichtung 16 durchgehend bis zu den Seitennahtbereichen 14 und definieren so eine in Hüftumfangsrichtung geschlossene Hüftöffnung 18 und Beinöffnungen 19, durch welche hindurch der Benutzer den Inkontinenzartikel wie ein Höschen anlegt.

Der Bauchabschnitt 4 lässt sich in einen hüftseitigen Bereich 20 und in einen schrittseitigen und den Beinöffnungen zugewandten Bereich 22 unterteilen. Eine entsprechende Unterteilung ist im Rückenabschnitt 6 vorgesehen, und zwar ebenfalls in einen hüftseitigen Bereich 24 und einen schrittseitigen und den Beinöffnungen zugewandten Bereich 26.

In dem hüftseitigen Bereich 20 des Bauchabschnitts 4 und in dem hüftseitigen Bereich 24 des Rükkenabschnitts 6 sind erste Elastifizierungsmittel 28 vorgesehen, bei denen es sich insbesondere um fadenförmige Elastifizierungsmittel, wie Lycra®-Fäden, handeln kann, die in vorgedehntem Zustand, im sogenannten Stretch-Bond-Verfahren, mit den Flachmaterialien (Chassismaterialien) des Bauchabschnitts 4 und des Rückenabschnitts 6 verbunden sind. Diese ersten Elastifizierungsmittel 28 erstrecken sich in Quer- oder Hüftumfangsrichtung 16 von einem Seitennahtbereich 14 zum anderen.

Der schrittseitige und den Beinöffnungen 19 zugewandte Abschnitt 22 des Bauchabschnitts 4 bzw. 26 des Rückenabschnitts 6 haben eine von der Quer- oder Hüftumfangsrichtung 16 abweichende in Richtung auf eine Quermittelachse 30 des Schrittabschnitts 8 zulaufende Randkontur 32 bzw. 34. Diese Randkontur 32, 34 ist auch in der Darstellung nach Figur 1 bogenförmig und daher zur Begrenzung der Beinöffnungen 19 geeignet. Durch diesen Verlauf des schrittseitigen und den Beinöffnungen zugewandten Bereichs 22 bzw. 26 wird auch ein verhältnismäßig großer Überlappungsbereich 36, 38 zwischen Schrittabschnitt 8 und Bauchabschnitt 4 bzw. Rückenabschnitt 6 realisiert, der im Hinblick auf eine reißfeste Verbindung von Schrittabschnitt 8 und Bauchabschnitt 4 bzw. Rückenabschnitt 6 wesentlich ist. Je größer der Überlappungsbereich 36, 38, umso geringere Klebermengen pro Fläche können verwendet werden, was sich im Hinblick auf die Steifigkeit der Chassismaterialien als vorteilhaft auswirkt. Insbesondere kann ein nicht vollflächiger Kleberauftrag zur Verbindung der Komponenten verwendet werden.

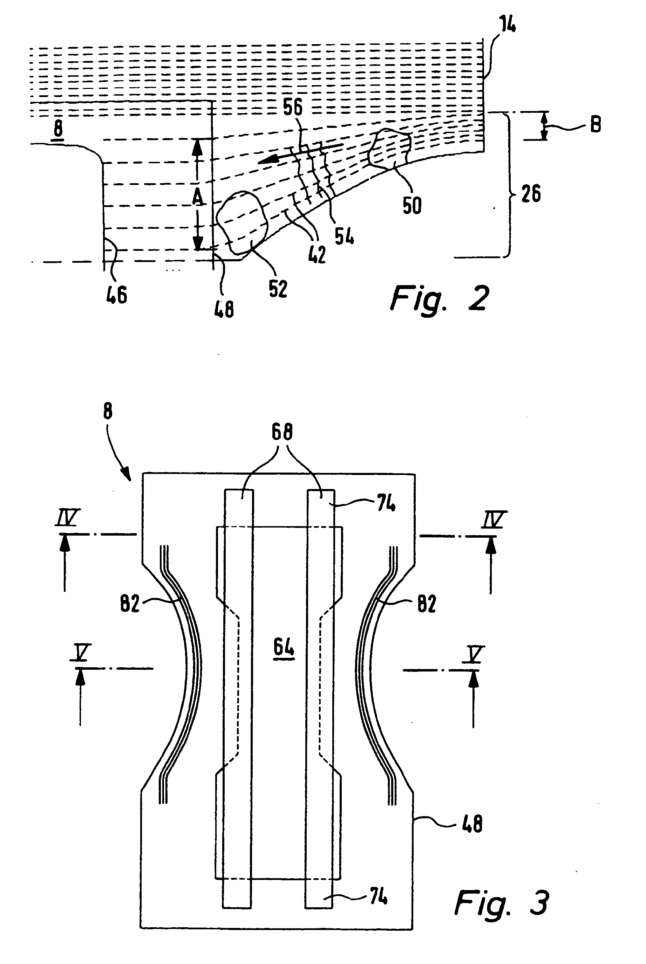

Der jeweilige schrittseitige und den Beinöffnungen 19 zugewandte Bereich 22, 26 des Bauchabschnitts 4 bzw. des Rückenabschnitts 6 ist ebenfalls elastifiziert ausgebildet. Dort sind zweite Elastifizierungsmittel 40 bzw. 42 vorgesehen. Die zweiten Elastifizierungsmittel 40, 42 erstrecken sich jeweils ausgehend von den Seitennahtbereichen 14 in Richtung auf eine Längsmittelachse 44 des Inkontinenzartikels. Wie aus den Figuren 1 und 2 zu erkennen ist, fächern die zweiten Elastifizierungsmittel 40, 42 in Richtung auf die Längsmittelachse 44 auf. Dies bedeutet, dass der Abstand zwischen ihnen in Richtung auf die Längsmittelachse 44 zunimmt. Diese Auffächerung der zweiten Elastifizierungsmittel 40 bzw.

42 lässt sich anhand Figur 2 auch quantitativ näher bezeichnen. Beispielsweise haben die in Figur 2 dargestellten zweiten Elastifizierungsmittel 42 des Rückenabschnitts 6 in den Seitennahtbereichen 14 einen minimalen Abstand von 3 bis 8 mm voneinander (Abstand von unmittelbar nebeneinander liegenden Elastifizierungsmitteln) und an einem Absorptionskörperrand 46 oder einem Längsrand 48 des Schrittabschnitts 8 einen maximalen Abstand (Abstand von unmittelbar nebeneinander liegenden Elastifizierungsmitteln) von 7 bis 35 mm voneinander. Es lässt sich anhand der Figur 2 auch ein Auffächerungsgrad F wie folgt definieren:

$$F = (A-B)/B * 100\%.$$

Dieser Auffächerungsgrad kann vorteilhafterweise zwischen 50 und 900 %, insbesondere zwischen 100 und 700 % und weiter insbesondere zwischen 150 bis 550 % liegen. Er ist im Rückenabschnitt 6 vorteilhafterweise größer als im Bauchabschnitt 4. Dabei sind die Größen A und B definiert als der Abstand des in Längsrichtung 9 äußersten zweiten Elastifizierungsmittels 40, 42 von dem in Längsrichtung 9 innersten zweiten Elastifizierungsmittel 40, 42 (also nicht der Abstand von unmittelbar nebeneinander liegenden zweiten Elastifizierungsmitteln), und zwar A als der maximale Abstand, insbesondere am Längsrand 48 des Schrittabschnitts 8, und B als der minimale Abstand, insbesondere im Seitennahtbereich 14.

Sofern der Auffächerungsgrad bei den zweiten Elastifizierungsmitteln 40, 42 hinreichend groß gewählt wird, so lässt sich auf diese Weise eine abnehmende Rückstellkraft innerhalb des schrittseitigen und den Beinöffnungen 19 zugewandten Bereichs 22 beziehungsweise 26 in Richtung 56 auf den Schrittabschnitt 8 realisieren, sofern dafür Sorge getragen wird, dass in Folge des von der Hüft- oder Querrichtung 16 abgewandten bogenförmigen Verlaufs der zweiten Elastifizierungsmittel 40, 42 sich keine zu starke Erhöhung der Vorspannung in Folge des größeren Wegs dieser zweiten Elastifizierungsmittel 40, 42 ergibt. Betrachtet man dann ein näher am Seitennahtbereich 14 liegendes Gebiet 50 des betreffenden schrittseitigen Bereichs 22 beziehungsweise 26 mit einem näher am Schrittabschnitt 8 liegenden Gebiet 52, so ist die Rückstellkraft, die sich bei flächenhafter Dehnung des Gebiets 52 (Dehnung in Richtung der Elastifizierungsmittel 42) einstellt geringer als diejenige Rückstellkraft, die sich bei Dehnung des Gebiets 50 einstellt. Dies führt in vorteilhafter Weise weiter dazu, dass in Folge der geringeren elastischen Kräfte, welche im beispielhaft dargestellten Fall durch die zweiten Elastizierungsmittel 40, 42 ausgeübt werden die Chassismaterialien des Bauchabschnitts 4 und des Rückenabschnitts 6 weniger stark gerafft werden, so dass sich auch eine geringere Anzahl von Falten/Rüschen 54 ergibt, und zwar ausgehend vom jeweiligen Seitennahtbereich 14 in Richtung auf den Schrittabschnitt 8. Dadurch, dass die sich bei flächenhafter Dehnung des Bauchabschnitts in dem schrittseitigen und den Beinöffnungen zugewandten Bereich 22 des Bauchabschnitts 4 beziehungsweise 26 des Rückenabschnitts 6 sich einstellenden Rückstellkräfte in Richtung des Pfeils 56, also generell vom Seitennahtbereich 14 in Richtung auf den Schrittabschnitt 8 abnehmen, wird eine erhebliche Verbesserung des Tragekomforts erzielt, weil gerade in diesen Bereichen - wie erfindungsgemäß festgestellt wurde - sich elastisch dehnbare Materialien als besonders problematisch erweisen, weil diese Materialien der Physiognomie der menschlichen Körperform entsprechend in diesen Bereichen besonders auf Zug und Dehnung beansprucht werden. Durch eine erfindungsgemäß bewusst vorgesehene Verringerung dieser Rückstellkraft, also abnehmende Rückstellkraft in Richtung des Pfeils 56, das heißt in Richtung zunehmender Annäherung an den Schrittabschnitt 8, wird hier ein zuvor nicht realisierter Freiheitsgrad geschaffen, mit dem die eingangs geschilderten Probleme überwunden werden.

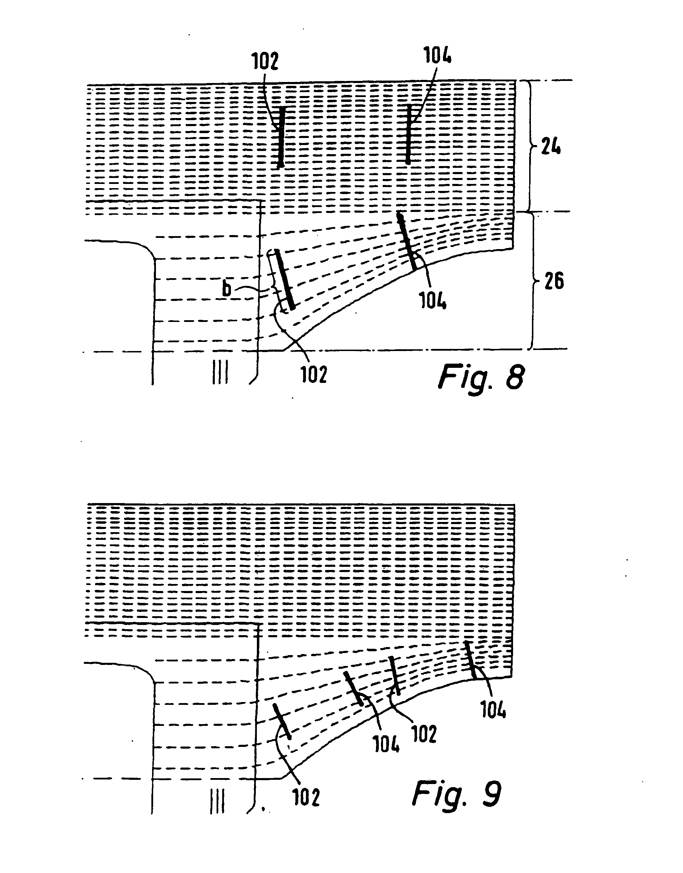

Wie eingangs ausgeführt, können Rückstellkräfte direkt am Chassis des Inkontinenzartikels bestimmt werden. Hierfür wird der betreffende Bereich des Bauchabschnitts 4 oder des Rückenabschnitts 6 zwischen zwei Klemmbacken 102, 104 (siehe Figur 8) von definierter, gleicher Klemmbackenbreite (b) eingespannt, und es werden dann Rückstellkräfte bei einer definierten, den Gebrauchszustand simulierenden Dehnung der zu messenden Bereiche um insbesondere 30% oder 50% oder 80% der Ausgangslänge (des Klemmbackenabstands im ungespannten Zustand) ermittelt. Die Klemmbacken 102, 104 werden dabei jeweils voneinander wegbewegt. Die Klemmbacken 102, 104 sollten möglichst viele, zumindest jedoch zwei nebeneinander angeordnete Elastifizierungsmittel 40, 42 bzw. 28 des zu messenden Bereichs fixieren, und sie sollten im Wesentlichen rechtwinklig zum Verlauf der Elastifizierungsmittel orientiert sein, so dass die Dehnung zwischen den Klemmbacken 102, 104, also die Auseinanderbewegung der Klemmbacken 102, 104, im Wesentlichen in Richtung des Verlaufs der Elastifizierungsmittel erfolgt. Dies ist in Figuren 8 und 9 verdeutlicht. Figur 8 zeigt prinzipiell die Anordnung von Klemmbacken 102, 104, um die Rückstellkräfte in dem schrittseitigen und den Beinöffnungen zugewandten Bereich 22, 26 mit den Rückstellkräften in einem hüftseitigen Bereich 20, 24 zu vergleichen. Figur 9 zeigt prinzipiell die Anordnung von Klemmbacken 102, 104, um die Rückstellkräfte in einem Gebiet näher am Schrittabschnitt 8 mit den Rückstellkräften in einem Gebiet näher am Seitennahtbereich 14 zu vergleichen.

Bei der dargestellten bevorzugten Ausführungsform des Inkontinenzartikels 2 beträgt ein Abstand C des schrittzugewandten innersten zweiten Elastifizierungsmittels 40 des Bauchabschnitts 4 von dem entsprechenden schrittzugewandten innersten zweiten Elastifizierungsmittel 42 des Rückenabschnitts 6 zwischen 250 bis 420 mm je nach Konfektionsgröße des Inkontinenzartikels. Die zweiten Elastifizierungsmittel 40, 42 erstrecken sich im Wesentlichen bis zum schrittzugewandten Querrand 58, 60 des Bauchabschnitts 4 und des Rückenabschnitts 6. Der Abstand von Bauchabschnitt 4 und Rückenabschnitt 6 in Längsrichtung (9) voneinander beträgt 250 - 400 mm.

Der Abstand der innersten, schrittzugewandten zweiten Elastifizierungsmittel 40, 42 von der die Beinöffnungen begrenzenden Randkontur 32, 34 des schrittseitigen und den Beinöffnungen zugewandten Bereichs 22, 26 des Bauchabschnitts 4 und des Rückenabschnitts 6 beträgt vorzugsweise 2 - 40 mm, weiter vorzugsweise 3 - 30 mm, insbesondere vorzugsweise 4 - 15 mm.

Die Erstreckung des Bauchabschnitts 4 und des Rückenabschnitts 6 im Seitennahtbereich 14 in Längsrichtung 9 beträgt vorteilhafterweise zwischen 100 und 220 mm. Die maximale Erstreckung des Schrittabschnitts 8 in Querrichtung 16 beträgt vorteilhafterweise 200 bis 350 mm.

Der Schrittabschnitt 8 umfasst ein flüssigkeitsundurchlässiges Backsheet-Material 62, das insbesondere von einem atmungsaktiven, jedoch flüssigkeitsdichten Folienmaterial gebildet sein kann, und ein, vorzugsweise auf Vliesbasis beruhendes Topsheet-Material 64. Zwischen dem Backsheet-Material und dem Topsheet-Material ist wie aus den Figuren 4, 5 ersichtlich der Absorptionskörper 7 angeordnet. Im beispielhaft dargestellten Fall bildet das Backsheet-Material 62 in Querrichtung 16 einen Überhang 66 über den Absorptionskörper 7. Das Topsheet 64 überragt den Absorptionskörper 7 in Querrichtung nur verhältnismäßig geringfügig; jedoch ist beidseits des Absorptionskörpers 7 in Längsrichtung 9 verlaufend jeweils ein aufstehendes Barrieremittel 68 vorgesehen, welches typischerweise als aufstehendes Cuffelement oder Bündchenelement bezeichnet wird und vorzugsweise aus einem hydrophoben, insbesondere flüssigkeitsundurchlässigen Vliesstoffmaterial gebildet ist, welches sich in Querrichtung 16 vorzugsweise bis zu den seitlichen Längsrändern 48 des Schrittabschnitts 8 erstreckt. Die distalen Enden 70 der Barrieremittel 68 sind mit weiteren Elastifizierungsmitteln 72 versehen, welche die Barrieremittel 68 im Gebrauch des Inkontinenzartikels gegen die Hautoberfläche des Benutzers anheben, so wie dies in Figur 5 schematisch dargestellt ist. An ihren jeweiligen Längsendbereichen 74 sind die seitlichen Barrieremittel 68 über schematisch angedeutete Fixierungen 76, 78 auf das Topsheet 64 beziehungsweise auf sich selbst in einer C-förmig gefalteten Konfiguration festgelegt. Vorteilhaft und erwähnenswert ist hier, dass die in der Figur 4 jeweils innenliegende Fixierung 78 das Barrieremittel 68 auf sich selbst festlegt, und zwar in Querrichtung 16 innerhalb der äußeren Fixierung 76, welche eine in Längsrichtung 9 durchgehend erstreckte Cuffsockellinie 80 bildet. Die innere Fixierung 78 ist hingegen nur in den Längsendbereichen 74 der Barrieremittel 68 vorgesehen.

Es erweist sich hier als besonders vorteilhaft, wenn der erwähnte Überhang 66 des Backsheet-Materials 62 und/oder des Topsheet-Materials 64 über den Absorptionskörper 7 - in der Summe beidseits des Absorptionskörpers, also in der Summe links und rechts - wenigstens 25 % bezogen auf die größte Breite des Schrittabschnitts 8 beträgt. Auf diese Weise ist nämlich in Querrichtung 16 Raum für die Anordnung entlang der Beinöffnungen 19 erstreckter Beinelastifizierungsmittel 82. Es erweist sich nämlich als vorteilhaft, wenn die Beinelastifizierungsmittel 82 mit einem gewissen Abstand vom materialreichen und damit eher biegesteifen Absorptionskörper 7 verlaufen, um einerseits keine zusätzlichen Dehnungs- oder Verwindungskräfte auf den Absorptionskörper auszuüben, was dessen Absorptionsverhalten nachteilig beeinflussen könnte, und um andererseits einen vom Absorptionskörper weitgehend unbeeinflussten flüssigkeitsdichten Beinabschluss zu realisieren. Es erweist sich im dargestellten Fall als besonders vorteilhaft, dass diese Beinelastifizierungsmittel 82 in Längsrichtung 9 mit einem deutlichen Abstand von insbesondere wenigstens 10 mm, vorzugsweise wenigstens 20 mm vor den zweiten Elastifizierungsmitteln 40 und 42 des Bauchabschnitts 4 beziehungsweise des Rückenabschnitts 6 enden. Vorzugsweise enden diese Beinelastifizierungsmittel 82 in Längsrichtung 9 vor dem Bauchabschnitt 4 und dem Rückenabschnitt 6. Dies ist deshalb vorteilhaft und wesentlich, weil die Beinelastifizierungsmittel 82 solchenfalls das Spannungsverhalten des Bauchabschnitts 4 und des Rückenabschnitts 6 wenig oder gar nicht beeinflussen. Es wurde nämlich erkannt, dass es sich im Hinblick auf das erfindungsgemäß zu erreichende Ziel der Verbesserung des Tragekomforts gerade in dem schrittseitigen und den Beinöffnungen 19 zugewandten Bereich 22 und 26 des Bauchabschnitts 4 und des Rückenabschnitts 6 als negativ erweist, wenn dort die üblicherweise mit großer Vorspannung und entsprechend großer Rückstellkraft ausgebildeten Beinelastifizierungsmittel 82 zusätzlich verlaufen.

Wie aus Figur 1 ersichtlich ist, ist bei dem Schrittabschnitt 8 ein in Querrichtung verhältnismäßig großer Überhang 66 über den Absorptionskörper 7 realisiert, und zwar insbesondere auch an dem Bauchabschnitt 4 beziehungsweise dem Rückenabschnitt 6 zugewandten Bereichen des Schrittabschnitts 8. Hierdurch wird - worauf bereits hingewiesen wurde - ein verhältnismäßig großer Überlappungsbereich 36, 38 des Schrittabschnitts 8 mit dem Bauchabschnitt 4 und mit dem Rückenabschnitt 6 realisiert. Nach einer bevorzugten Ausführungsvariante umfasst der Überlappungsbereich 36 von Schrittabschnitt 8 mit dem Bauchabschnitt 4 wenigstens 12% der Fläche des Bauchabschnitts 4, und der Überlappungsbereich 38 von Schrittabschnitt 8 mit dem Rückenabschnitt 6 umfasst wenigstens 20% der Fläche des Rückenabschnitts 6. Dies erweist sich als vorteilhaft, da solchenfalls eine sichere Fixierung des Schrittabschnitts 8 an dem Bauchabschnitt 4 beziehungsweise an dem Rückenabschnitt 6 erreicht werden kann, und zwar auch, wenn kein vollflächiger Kleberauftrag verwendet wird. Es ist solchenfalls in vorteilhafter Weise hinreichend, wenn ein nur abschnittsweiser oder gerasterter Kleberauftrag zur Verbindung verwendet wird. Dies ist deshalb vorteilhaft, weil solchenfalls die miteinander gefügten Materialien nicht zu steif werden.

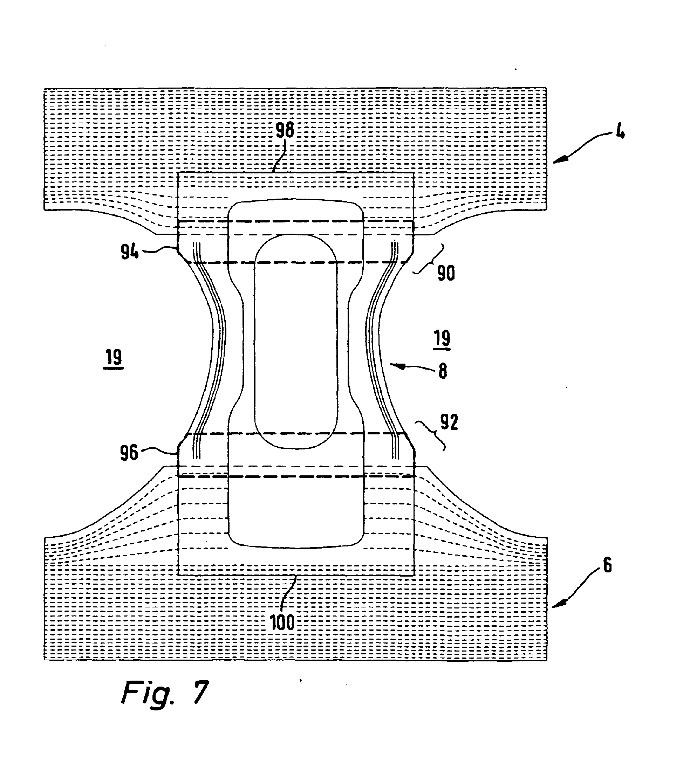

Anhand der Figur 7, die der Figur 1 entspricht, wird ein weiteres vorteilhaftes Detail des erfindungsgemäßen Inkontinenzartikels erläutert. Durch die Verfolgung des dreikomponentigen Konzepts zur Herstellung des erfindungsgemäßen Inkontinenzartikels ergibt sich ein Übergang 90 zwischen Schrittabschnitt 8 und Bauchabschnitt 4 sowie ein Übergang 92 zwischen Schrittabschnitt 8 und Rückenabschnitt 6, bei denen sich üblicherweise ein unstetiger, also mit Ecken oder Winkeln oder Knicken versehener Verlauf der die Beinöffnungen 19 begrenzenden Ränder der Chassismaterialien ergibt. Dies birgt die Gefahr, dass im Bereich der Übergänge 90, 92 Kraftspitzen gebildet werden, die zu einem Einreißen der Chassismaterialien führen können, was die Anfügung des Schrittabschnitts 8 an den Bauchabschnitt 4 beziehungsweise an den Rückenabschnitt 6 beeinträchtigen kann. Um dem entgegen zu wirken ist im jeweiligen Übergang 90 und 92 eine verstärkende Beschichtung 94, 96 des flüssigkeitsundurchlässigen Backsheet-Materials 62 des Schrittabschnitts 8 vorgesehen. Es erweist sich als hinreichend, wenn diese verstärkende Beschichtung 94, 96 nur in dem durch die unterbrochene Linie jeweils bezeichneten Bereich der Figur 7 vorgesehen wird. Die verstärkende Beschichtung 94, 96 überlappt im beispielhaft und vorzugsweise dargestellten Fall den Bauchabschnitt 4 und den Rückenabschnitt 6 in Längsrichtung 9 lediglich um etwa 10 bis 20 mm, insbesondere um ca. 15 mm. Die verstärkende Beschichtung endet in Längsrichtung 9 jeweils vor den Längsenden 98, 100 des Schrittabschnitts, und zwar wenigstens 30 mm vor dem bauchseitigen Längsende 98 und wenigstens 90 mm vor dem rückenseitigen Längsende 100. Dies erweist sich als vorteilhaft, da solchenfalls die verstärkende Beschichtung 94, 96 nicht unnötig zur Versteifung der Chassismaterialien in Bereichen beiträgt, wo dies nicht hilfreich, sondern eher unerwünscht und nachteilig ist. Außerdem können auf diese Weise Materialkosten gespart werden. Jedoch bleibt die Möglichkeit unberührt, die verstärkende Beschichtung 94, 96 nicht nur im Übergang 90 beziehungsweise 92 vorzusehen.

Die verstärkende Beschichtung 94, 96 besteht vorzugsweise aus einem Spinnvlies aus Polypropylen mit einem Flächengewicht von 10 - 20 g/m$^2$, insbesondere von 12 -17 g/m$^2$.

Erfindungsgemäßes Verfahren:

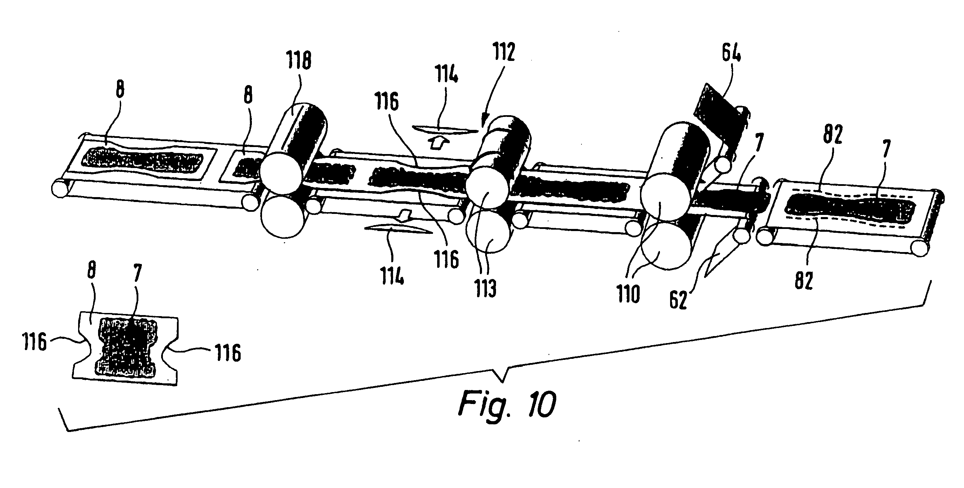

Die Figuren 10 bis 13 zeigen das erfindungsgemäße Herstellungsverfahren. Figur 10 zeigt die Herstellung von Schrittabschnitten 8. Hierfür werden einer schnell laufenden Herstellungsmaschine eine endlose Backsheet-Materialbahn 62 und eine endlose Topsheet-Materialbahn 64 und aufeinander folgend Absorptionskörper 7 sowie (nur angedeutet) den späteren Beinöffnungen zugeordnete Beinelastifizierungsmittel 82 zugeführt. Die Absorptionskörper 7 werden voneinander beabstandet zwischen die Topsheet-Materialbahn 64 und die Backsheet-Materialbahn 62 angeordnet, und der so gebildete Verbund wird durch geeignete Fügemittel, insbesondere Kleber, fixiert. Auch die Beinelastifizierungsmittel 82 werden zwischen die Topsheet-Materialbahn 64 und die Backsheet-Materialbahn 62 zugeführt und fixiert. Hierfür wird ein erstes Walzenpaar 110 und in nicht dargestellter Weise ein Kleber verwendet. Der Vollständigkeit halber sei erwähnt, dass der Schrittabschnitt 8 zusätzlich seitliche aufstehende und vorzugsweise elastifizierte Cuffs als Seitenauslaufschutz umfasst. Diese sind vorliegend auf der körperzugewandten Seite der Topsheet-Materialbahn 64 bereits vorgesehen jedoch nicht dargestellt; sie könnten aber auch an beliebiger Stelle des in Figur 10 dargestellten Fertigungsablaufs oder auch an späterer Stelle eingebracht werden.

In Förderrichtung an das vorstehend Beschriebene anschließend wird in dem Verbund ein erster Konturschnitt 112 unter Verwendung eines Schneidwalzenpaars 113 ausgeführt. Im Zuge des Konturschnitts 112 werden von dem den Schrittabschnitt 8 bildenden Verbund aus Backsheet-Materialbahn 62 und Topsheet-Materialbahn 64 bogenförmige Segmente 114 ausgeschnitten, um so Beinausschnitte 116 bei dem Schrittabschnitt 8 zu bilden. Der Verbund wird dann zu einer nachfolgenden Schneidstation 118 gefördert, und dort wird quer zur Förderrichtung ein Trennschnitt zur Vereinzelung der Schrittabschnitte 8 des herzustellenden Inkontinenzartikels ausgeführt. Ebenfalls in Figur 10 angedeutet ist eine perspektivische Ansicht des Schrittabschnitts 8 mit Absorptionskörper 7 und Beinausschnitten 116.

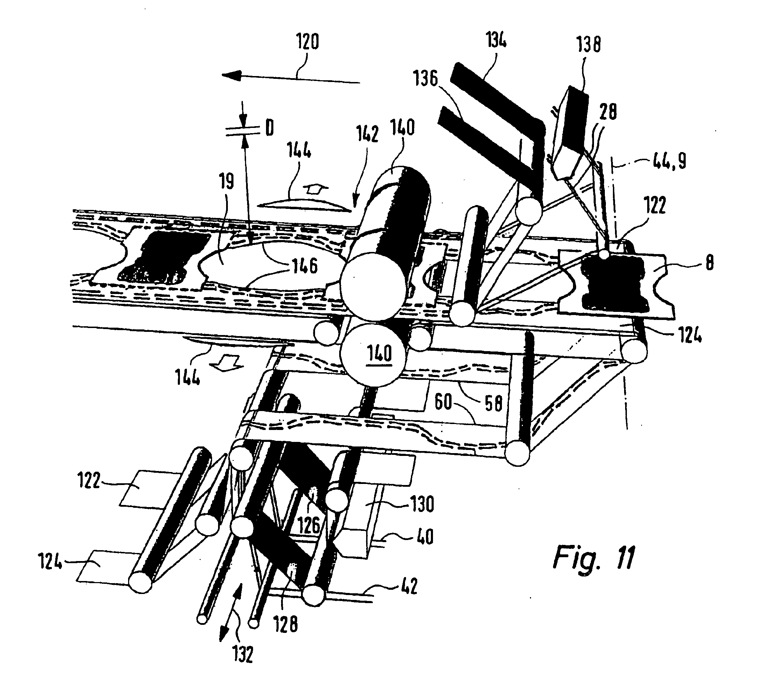

Nach dem Vereinzeln der Schrittabschnitte 8 werden diese im Zuge der weiteren Förderung um 90° gedreht und dann quer zur späteren Längsmittelachse 44 des Inkontinenzartikels 2 in der Maschinenrichtung 120 weitergefördert(Figur 11). Wie ebenfalls aus Figur 11 ersichtlich ist, werden zur Herstellung des späteren Bauchabschnitts 4 und Rückenabschnitts 6 des Inkontinenzartikels Teilbahnen 122, 124 auf Vliesbasis zugeführt. Diese Teilbahnen 122, 124 können ausgehend von einer einzigen Bahn durch Trennen in Längsrichtung gebildet sein. Auf diese Teilbahnen 122, 124 werden die eingangs erwähnten zweiten Elastifizierungsmittel 40 und 42 aufgebracht, die hierfür ebenfalls endlos und in Förderrichtung der Teilbahnen 122, 124 zugeführt werden. Zur Fixierung der zweiten Elastifizierungsmittel 40, 42 auf den Teilbahnen 122, 124 wird jeweils eine Vliesdeckschicht 126, 128, die zuvor in einer Beleimungsstation 130 mit Kleber beaufschlagt wurde, aufgebracht, so dass die zweiten Elastifizierungsmittel 40, 42 zwischen den Teilbahnen 122, 124 und den Vliesdeckschichten 126, 128 einlaminiert werden. Obschon aus Figur 11 infolge der schematischen Darstellung nicht ersichtlich werden die zweiten Elastifizierungsmittel 40, 42 in einem variierenden Abstand voneinander zugeführt, was durch eine oszillierende Führungseinrichtung, die durch den Doppelpfeil 132 angedeutet ist, realisiert wird. So wird durch entsprechende Ansteuerung der Führungseinrichtung für jedes einzelne der Elastifizierungsmittel 40, 42 der bogenförmige sich auffächernde Verlauf der zweiten Elastifizierungsmittel 40, 42 in Richtung auf den Schrittabschnitt 8 gebildet.

Die Teilbahnen 122, 124 werden dann in dem beschriebenen Verbund weitergefördert und mit den Schrittabschnitten 8 zusammengeführt, und zwar derart, dass die Schrittabschnitte in einer Längsrichtung 9 quer zur Maschinenrichtung 120 einenends mit der einen Teilbahn 122 und anderenends mit der anderen Teilbahn 124 überlappen. Die Schrittabschnitte 8 werden so zugeführt, dass sie nach dem Zusammenführen mit einem Abstand in der Maschinenrichtung 120 voneinander angeordnet sind. Die Schrittabschnitte 8 und die Teilbahnen 122, 124 werden in der aus Figur 11 erhaltenen Konfiguration miteinander fixiert und in der Maschinenrichtung 120 weitergefördert.

Es werden dann die in Quer- oder Hüftumfangsrichtung 16 erstreckten ersten Elastifizierungsmittel 28 endlos in der Maschinenrichtung 120 zugeführt und auf den Teilbahnen 122, 124 fixiert. Es werden wiederum Vliesbahnen 134, 136 zugeführt. Jedoch werden die Vliesbahnen 134, 136 nicht direkt mit Kleber versehen, sondern der Kleber wird auf die ersten Elastifizierungsmittel 28 aufgebracht, und die ersten Elastifizierungsmittel 28 werden dann auf die Teilbahnen 122, 124 aufgebracht und von den Vliesbahnen 134, 136 überdeckt, so dass sie einlaminiert werden.

Es wäre aber möglicherweise auch denkbar, dass die Elastifizierungsmittel 28 und 40, 42 alle einzeln mit Kleber versehen werden, also fadenbeleimt werden.

Es wäre möglicherweise auch denkbar, dass unabhängig von der Art des Klebemittelauftrags auf die Elastifizierungsmittel 28 und 40, 42 auf die Vliesdeckschichten 126, 128, 134 und/oder 136 verzichtet werden kann. Allerdings haben die Vliesdeckschichten den Vorteil, dass sie zugleich eine weich anfühlende Innenseite des Inkontinenzartikels bilden.

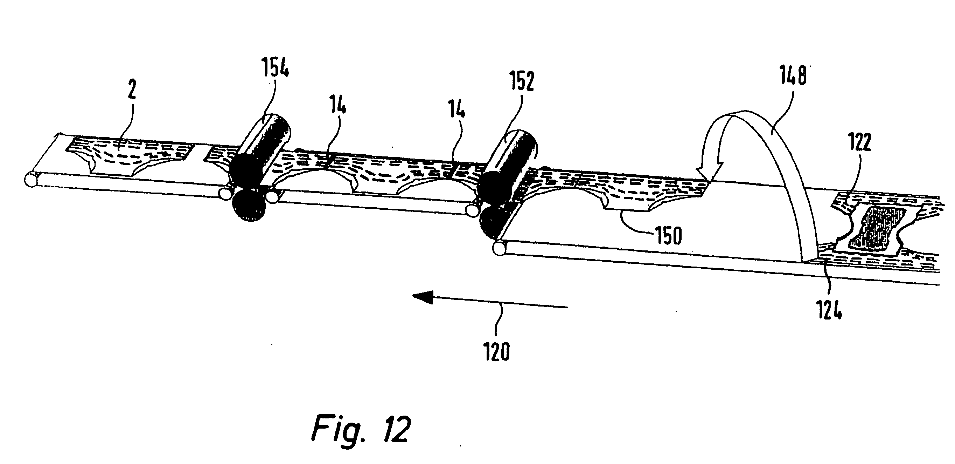

Dem nachfolgend ist in Figur 11 ein weiteres Schneidwalzenpaar 140, also ein Rotationsmesser mit einer Ambosswalze, vorgesehen, zwischen denen der bislang gebildete Verbund in der Maschinenrichtung 120 in der beschriebenen Orientierung hindurchgeführt wird. Dabei wird ein zweiter Konturschnitt 142 ausgeführt, im Zuge dessen, vorzugsweise nur von den Teilbahnen 122, 124, je ein bogenförmiges Segment 144 abgetrennt wird, und zwar von den einander zugewandten Querrändern oder Randabschnitten 58 und 60 der Teilbahnen 122, 124, so dass Beinausschnitte 146 auch bei den Teilbahnen 122, 124 gebildet werden. Dadurch, dass der zweite Konturschnitt 142 nicht den Schrittabschnitt 8 sondern nur die Teilbahnen 122, 124 erfasst, verläuft der zweite Konturschnitt 142 im Wesentlichen entlang der Maschinenrichtung 120 und jedenfalls nicht unter großem Winkel quer hierzu. Auf diese Weise kann der Schnitt optimal konfiguriert werden, ebenso wie der erste Konturschnitt 112 im Zuge der Herstellung des Schrittabschnitts 8. Insgesamt können so die späteren Beinöffnungen 19 des Inkontinenzartikels 2 mit hoher Genauigkeit den als optimal erachteten Anforderungen entsprechend ausgebildet werden. Dabei erweist es sich als vorteilhaft, dass der zweite Konturschnitt 142 an der Teilbahn 122 mit einem anderen Verlauf ausgebildet sein kann als an der Teilbahn 124. So kann die Gestalt der Beinausschnitte 146 bzw. der späteren Beinöffnungen 19 des Inkontinenzartikels 2 im Bauchabschnitt 4 und im Rückenabschnitt 6 verschieden konfiguriert werden. Der so gebildete Verbund wird weitergefördert, und in einer in Figur 12 nur angedeuteten Faltstation 148 wird der Verbund um eine in Maschinenrichtung 120 verlaufende Faltlinie 150 auf sich selbst gefaltet, derart, dass die eine Teilbahn 124 über die andere Teilbahn 122 zu liegen kommt. Daran anschließend wird in einer Fügestation 152 ein jeweiliger Seitennahtbereich 14 zwischen den Teilbahnen 122, 124 ausgebildet, es wird also die eigentlichen Höschenform gebildet. An diesen Verfahrensschritt anschließend wird in einer Trennstation 154 ein Trennschnitt quer zur Maschinenrichtung 120 ausgeführt, der zur Vereinzelung der fertigen Inkontinenzartikel 2 führt. Es wäre auch denkbar, dass die Fügestation 152 zugleich als Trennstation ausgebildet ist, beispielsweise in Form einer Trennschweißeinrichtung, so dass zusammen mit der Ausbildung des Seitennahtbereichs 14 die Inkontinenzartikel 2 vereinzelt werden.

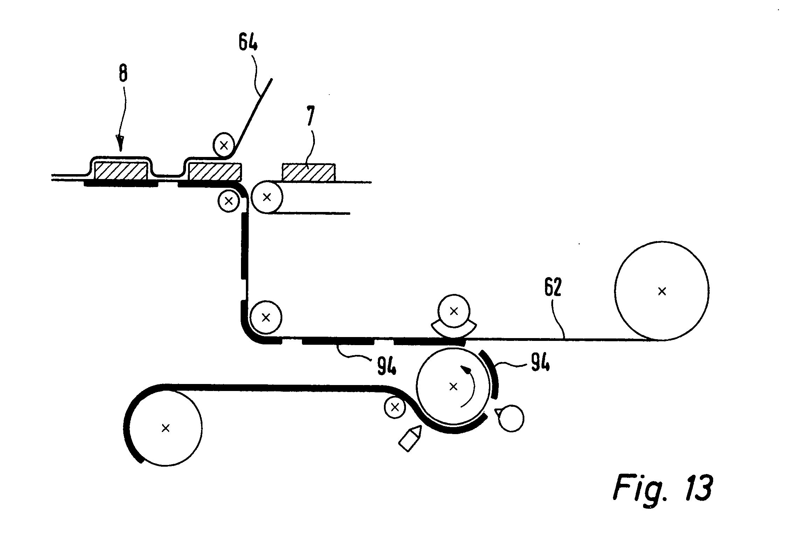

Schließlich zeigt Figur 13 schematisch eine Verfahrensführung, bei der die Backsheet-Materialbahn 62 abschnittsweise mit einer im Zusammenhang mit Figur 7 erläuterten verstärkenden Beschichtung 94 versehen wird, und zwar im Zuge der Zuführung der Backsheet-Materialbahn 62 zur Herstellung der Schrittabschnitte, was bereits im Zusammenhang mit Figur 10 erläutert wurde. Im Unterschied zu Figur 8 überfängt die verstärkende Beschichtung 94, bei der es sich insbesondere um einen Vliesabschnitt handeln kann, im Wesentlichen einen Großteil des Schrittabschnitts 8; an den Längsenden des zu bildenden Schrittabschnitts 8 bleibt die Backsheetmaterialbahn 62 aber unbeschichtet.

**Patentansprüche**

1. Kontinuierliches Verfahren zum Herstellen von Inkontinenzartikeln (2) in Höschenform für die Aufnahme von Körperausscheidungen, mit einem vorderen Bauchabschnitt (4) und einem hinteren Rückenabschnitt (6), die zur Bildung eines in Quer- oder Hüftumfangsrichtung (16) durchgehenden Bauch- und Rückenbands mit einer in Hüftumfangsrichtung (16) geschlossenen Hüftöffnung (18) an beidseitigen Seitennahtbereichen (14) herstellerseitig miteinander verbunden sind, und mit einem einen Absorptionskörper (7) aufweisenden Schrittabschnitt (8), der in einer Längsrichtung (9) zwischen Bauchabschnitt (4) und Rückenabschnitt (6) erstreckt und an den Bauchabschnitt (4) und an den Rückenabschnitt (6) unlösbar angefügt ist, wobei sowohl der Schrittabschnitt (8) als auch der Bauchabschnitt (4) und der Rückenabschnitt (6) die Beinöffnungen (19) des Inkontinenzartikels (2) begrenzen, wobei in dem Bauchabschnitt (4) und dem Rückenabschnitt (6) erste Elastifizierungsmittel (28) vorgesehen sind, die sich in einem Abstand voneinander und parallel zueinander in Quer- oder Hüftumfangsrichtung (16) erstrecken und so den Bauchbereich (4) und den Rückenbereich (6) flächenhaft elastifizieren, wobei in einem schrittseitigen und den Beinöffnungen (19) zugewandten Bereich (22, 26) des Bauchabschnitts (4) und des Rückenabschnitts (6) zweite Elastifizierungsmittel (40, 42) vorgesehen sind, die sich ausgehend von den beiden Seitennahtbereichen (14) in Richtung (56) auf eine Längsmittelachse (44) des Inkontinenzartikels (2) erstrecken und dabei bogenförmig sich auffächernd mit zunehmendem Abstand voneinander verlaufen, die folgenden Verfahrensschritte umfassend:

- Zuführen von Absorptionskörpern (7) aufweisenden Schrittabschnitten (8), welche durch einen ersten Konturschnitt (112) gebildete Beinausschnitte (116) aufweisen,
- Zuführen zweier den späteren Bauchabschnitt (4) bzw. den späteren Rückenabschnitt (6) des Inkontinenzartikels (2) bildenden Teilbahnen (122, 124) auf Vliesbasis,
- Zuführen und Aufbringen der zweiten Elastifizierungsmittel (40, 42) auf die beiden Teilbahnen (122, 124) und Fixieren daran,
- Zusammenführen der Schrittabschnitte (8) mit den beiden Teilbahnen (122, 124), derart, dass die Schrittabschnitte (8) in einer Längsrichtung (9) quer zur Maschinenrichtung (120) einenends mit der einen Teilbahn (122) und anderenends mit der anderen Teilbahn (124) überlappen und die Schrittabschnitte (8) mit einem Abstand in der Maschinenrichtung (120) voneinander angeordnet sind, und Fixieren der Schrittabschnitte (8) und Teilbahnen (122, 124) in den Überlappungsbereichen, und Weiterfördern in der Maschinenrichtung (120),
- Zuführen, Aufbringen und Fixieren der ersten Elastifizierungsmittel (28) in der Maschinenrichtung (120) an den Teilbahnen (122, 124),
- Ausführen eines die Teilbahnen (122, 124) an ihren einander zugewandten Randabschnitten (58, 60) erfassenden zweiten Konturschnitts (142) zur Bildung von im Wesentlichen bogenförmigen Beinausschnitten (146),
- Falten um eine in Maschinenrichtung (120) verlaufende Faltlinie (150), derart, dass die eine Teilbahn (122) über die andere Teilbahn (124) zu liegen kommt,
- Fügen der übereinander liegenden Teilbahnen (122, 124) quer zur Maschinenrichtung (120) in Abständen voneinander zur Ausbildung von Seitennahtbereichen (14) der herzustellenden Inkontinenzartikel (2), und Erhalt von einen Bauchabschnitt (4), einen Rückenabschnitt (6) und einen dazwischen angeordneten Schrittabschnitt (8) aufweisenden Produkten,

- Ausführen eines Trennschnitts quer zur Maschinenrichtung (120) und Erhalt von vereinzelten fertigen Inkontinenzartikeln (2).

2. Verfahren nach Anspruch 1, **dadurch gekennzeichnet, dass** nach dem Verfahrensschritt des Zuführens, Aufbringens und Fixierens der zweiten Elastifizierungsmittel (40, 42) eine die zweiten Elastifizierungsmittel (40, 42) überfangende Vliesdeckschicht (126, 128) auf jede Teilbahn (122, 124) zugeführt und aufgebracht wird und die zwei Teilbahnen (122, 124) in der Maschinenrichtung in einem Abstand voneinander und parallel zueinander weitergefördert werden.

3. Verfahren nach Anspruch 1 oder 2, **dadurch gekennzeichnet, dass** nach dem Verfahrensschritt des Zuführens, Aufbringens und Fixierens der ersten Elastifizierungsmittel (28) eine die ersten Elastifizierungsmittel überfangende Vliesdeckschicht (134, 136) auf jede Teilbahn (122, 124) zugeführt und aufgebracht wird und die zwei Teilbahnen (122, 124) in der Maschinenrichtung in einem Abstand voneinander und parallel zueinander weitergefördert werden.

4. Verfahren nach Anspruch 1, 2 oder 3, **dadurch gekennzeichnet, dass** der erste Konturschnitt (112) zur Bildung der Beinausschnitte (116) bei dem Schrittabschnitt (8) innerhalb des kontinuierlichen Verfahrens ausgeführt wird.

5. Verfahren nach Anspruch 4, **dadurch gekennzeichnet, dass** die Schrittabschnitte (8) innerhalb des kontinuierlichen Verfahrens gebildet werden, indem eine endlose Topsheet-Materialbahn (64), eine endlose Backsheet-Materialbahn (62) und Absorptionskörper (7) in einer Maschinenrichtung zugeführt und die Absorptionskörper (7) voneinander beabstandet zwischen die Topsheet-Materialbahn (64) und die Backsheet-Materialbahn (62) angeordnet werden und der so gebildete Verbund in sich fixiert wird und dass in dem Verbund der erste Konturschnitt (112) ausgeführt wird, um die Beinausschnitte (116) bei den Schrittabschnitten (8) zu bilden.

6. Verfahren nach einem oder mehreren der vorstehenden Ansprüche, **dadurch gekennzeichnet, dass** den Schrittabschnitten (8) Beinelastifizierungsmittel (82) zugeführt werden, insbesondere zwischen Topsheet-Materialbahn (64) und Backsheet-Materialbahn (62).

7. Verfahren nach Anspruch 6, **dadurch gekennzeichnet, dass** die Beinelastifizierungsmittel (82) derart zugeführt werden, dass sie mit veränderlichem Abstand zum Absorptionskörper (7) verlaufen und an ihren Längsenden einen größeren Abstand zum Absorptionskörper (7) aufweisen als mittig

8. Verfahren nach Anspruch 5, 6 oder 7 , **dadurch gekennzeichnet, dass** die Backsheet-Materialbahn (62) in Form einer flüssigkeitsundurchlässigen Folienbahn zugeführt wird, die mit einer Vliesbeschichtung und/oder einer verstärkenden Beschichtung (94) versehen wird.

9. Verfahren nach einem oder mehreren der vorstehenden Ansprüche, **dadurch gekennzeichnet, dass** zunächst eine endlose Vliesbahn zugeführt wird, die dann zur Bildung der zwei Teilbahnen (122, 124) entlang der Maschinenrichtung aufgetrennt wird.

10. Verfahren nach einem oder mehreren der vorstehenden Ansprüche, **dadurch gekennzeichnet, dass** die in Hüftumfangsrichtung (16) verlaufenden ersten Elastifizierungsmittel (28) in einem Abstand von 4 - 10 mm, insbesondere 4 - 8 mm, insbesondere 4 - 6 mm voneinander eingebracht werden.

11. Verfahren nach einem oder mehreren der vorstehenden Ansprüche, **dadurch gekennzeichnet, dass** die zweiten Elastifizierungsmittel (40, 42) in einem variierenden Abstand voneinander zwischen 3 mm und 35 mm eingebracht werden.

12. Verfahren nach einem oder mehreren der vorstehenden Ansprüche, **dadurch gekennzeichnet, dass** die ersten und/oder zweiten Elastifizierungsmittel (28, 40, 42) unter einer Vorspannung von 1,5 - 6,0, insbesondere von 2,5 - 5,0 an den Teilbahnen fixiert werden.

13. Verfahren nach einem oder mehreren der vorstehenden Ansprüche, **dadurch gekennzeichnet, dass** die zweiten Elastifizierungsmittel (40, 42) in einem Überlappungsbereich mit dem Absorptionskörper (7) deaktiviert werden.

14. Verfahren nach einem oder mehreren der vorstehenden Ansprüche, **dadurch gekennzeichnet, dass** der zweite Konturschnitt (142) entlang und im Abstand (D) zu dem in Längsrichtung jeweils innersten der schrittzugewandten zweiten Elastifizierungsmittel (40, 42) ausgeführt wird.

15. Verfahren nach Anspruch 14, **dadurch gekennzeichnet, dass** der Abstand (D) 2 - 40 mm, insbesondere 3 - 30 mm und weiter insbesondere 4 - 20 mm beträgt.

**16.** Verfahren nach einem oder mehreren der vorstehenden Ansprüche, **dadurch gekennzeichnet, dass** der zweite Konturschnitt (142) nur die Teilbahnen (122, 124) nicht aber den Schrittabschnitt (8) erfasst.

**17.** Verfahren nach einem oder mehreren der vorstehenden Ansprüche, **dadurch gekennzeichnet, dass** der bogenförmige Beinausschnitt (146) der Teilbahn (122, 124) des späteren Bauchabschnitts (4) einen anderen Verlauf, insbesondere einen anderen Winkel oder Radius, aufweist als der bogenförmige Beinausschnitt (146) der Teilbahn (122, 124) des späteren Rückenabschnitts (6).

**18.** Verfahren nach einem oder mehreren der vorstehenden Ansprüche, **dadurch gekennzeichnet, dass** entlang der Beinöffnungen (19) ein unstetiger Verlauf im Übergang von Schrittabschnitt (8) zu Bauchabschnitt (4) und/oder Rückenabschnitt (6) vorgesehen ist.

**19.** Verfahren nach einem oder mehreren der vorstehenden Ansprüche, **dadurch gekennzeichnet, dass** das Fügen der übereinander liegenden Teilbahnen (122, 124) zur Ausbildung von Seitennahtbereichen (14) der herzustellenden Inkontinenzartikel und das Ausführen des Trennschnitts in demselben Verfahrensschritt durchgeführt werden.

## Claims

**1.** Continuous method for producing incontinence articles (2) in the form of pants for receiving body excrements, comprising a front belly section (4) and a rear back section (6), which are connected to each other at side seam areas (14) on both sides during production to form a belly and back band which is continuous in the transverse or peripheral hip direction (16) and has a hip opening (18) which is closed in the peripheral hip direction (16), and a crotch section (8) which comprises an absorbent body (7) and extends in a longitudinal direction (9) between the belly section (4) and the back section (6) and is permanently attached to the belly section (4) and the back section (6), wherein the crotch section (8) as well as the belly section (4) and the back section (6) define leg openings (19) of the incontinence article (2), wherein first elastification means (28) are provided in the belly section (4) and the back section (6), which extend at a separation from each other and parallel to each other in the transverse or peripheral hip direction (16) and thereby extensively elasticise the belly section (4) and the back section (6), wherein, in an area (22, 26) of the belly section (4) and the back section (6) on the crotch side facing the leg openings (19), second elastification means (40, 42) are provided, which extend starting from the two side seam areas (14) in the direction (56) towards a longitudinal center axis (44) of the incontinence article (2) in a curved shape, thereby fanning out with increasing separation from each other, comprising the following method steps:

- supplying crotch sections (8) which comprise absorbent bodies (7) and leg cut-outs (116) formed by a first contour cut (112);
- supplying two partial webs (122, 124) based on non-woven material, which form the subsequent belly section (4) and the subsequent back section (6) of the incontinence article (2);
- supplying and applying the second elastification means (40, 42) to both partial webs (122, 124) and fixing them thereto;
- bringing together the crotch sections (8) with the two partial webs (122, 124) in such a fashion that one end of the crotch sections (8) overlaps one partial web (122) in a longitudinal direction (9) transversely to the machine direction (120), and the other end thereof overlaps the other partial web (124), and the crotch sections (8) are disposed at a separation from each other in the machine direction (120), fixing the crotch sections (8) and partial webs (122, 124) in the overlapping areas, and forwarding in the machine direction (120);
- supplying, applying and fixing the first elastification means (28) to the partial webs (122, 124) in the machine direction (120);
- performing a second contour cut (142) that includes the partial webs (122, 124) on their mutually facing edge sections (58, 60) for forming substantially curved leg cut-outs (146);
- folding about a folding line (150) that extends in the machine direction (120), in such a fashion that one partial web (122) comes to rest on top of the other partial web (124);
- joining the partial webs (122, 124), which are disposed on top of each other, transversely to the machine direction (120) at a separation from each other for forming side seam areas (14) of the incontinence articles (2) to be produced, and obtaining products which comprise a belly section (4), a back section (6), and an intermediate crotch section (8);
- performing a separating cut in a direction transversely to the machine direction (120) and obtaining separated finished incontinence articles (2).

**2.** Method according to claim 1, **characterized in that** after the method step of supplying, disposing and fixing the second elastification means (40, 42), a non-woven cover layer (126, 128), which extends over the second elastification means (40, 42), is supplied to and disposed on each partial web (122, 124), and the two partial webs (122, 124) are further transported in the machine direction at a separation from each other and parallel to each other.

3. Method according to claim 1 or 2, **characterized in that** after the method step of supplying, disposing and fixing the first elastification means (28), a non-woven cover layer (134, 136), which extends over the first elastification means, is supplied to and disposed on each partial web (12, 124), and the two partial webs (122, 124) are further transported in the machine direction at a separation from each other and parallel to each other.

4. Method according to claim 1, 2, or 3, **characterized in that** the first contour cut (112) for forming the leg cut-outs (116) of the crotch section (8) is performed within the continuous method.

5. Method according to claim 4, **characterized in that** the crotch sections (8) are formed within the continuous method **in that** an endless topsheet material web (64), an endless backsheet material web (62), and absorbent bodies (7) are supplied in a machine direction, the absorbent bodies (7) are disposed at a separation from each other between the topsheet material web (64) and the backsheet material web (62), the composite formed in this fashion is secured, and the first contour cut (112) is performed in the composite in order to form the leg cut-outs (116) of the crotch sections (8).

6. Method according to any one or more of the preceding claims, **characterized in that** leg elastification means (82) are supplied to the crotch sections (8), in particular between the topsheet material web (64) and the backsheet material web (62).

7. Method according to claim 6, **characterized in that** the leg elastification means (82) are supplied in such a fashion that they extend at a varying separation from the absorbent body (7) and have a larger separation from the absorbent body (7) at their longitudinal ends than in the center.

8. Method according to claim 5, 6, or 7, **characterized in that** the backsheet material web (62) is supplied in the form of a liquid-impermeable film web that is provided with a non-woven coating and/or a reinforcing coating (94).

9. Method according to any one or more of the preceding claims, **characterized in that** an endless non-woven material web is initially supplied, which is then separated along the machine direction to form the two partial webs (122, 124).

10. Method according to any one or more of the preceding claims, **characterized in that** the first elastification means (28), which extend in the peripheral hip direction (16), are introduced at a separation from each other of 4 to 10 mm, in particular 4 to 8 mm, in particular 4 to 6 mm.

11. Method according to any one or more of the preceding claims, **characterized in that** the second elastification means (40, 42) are introduced at a varying distance from each other of between 3 mm and 35 mm.

12. Method according to any one or more of the preceding claims, **characterized in that** the first and/or second elastification means (28, 40, 42) are fixed to the partial webs under a pretension of 1.5 to 6.0, in particular 2.5 to 5.0.

13. Method according to any one or more of the preceding claims, **characterized in that** the second elastification means (40, 42) are deactivated in an overlapping area with the absorbent body (7).

14. Method according to any one or more of the preceding claims, **characterized in that** the second contour cut (142) is performed along and at a separation (D) in the longitudinal direction from the respective innermost second elastification means (40, 42) facing the crotch area.

15. Method according to claim 14, **characterized in that** the separation (D) is 2 to 40 mm, in particular 3 to 30 mm and, in particular 4 to 20 mm.

16. Method according to any one or more of the preceding claims, **characterized in that** the second contour cut (142) only includes the partial webs (122, 124) and not the crotch section (8).

17. Method according to any one or more of the preceding claims, **characterized in that** the curved leg cut-out (146) of the partial web (122, 124) of the subsequent belly section (4) extends in a different shape, in particular at a different angle or radius, than the curved leg cut-out (146) of the partial web (122, 124) of the subsequent back section (6).

18. Method according to any one or more of the preceding claims, **characterized in that** the transition between the crotch section (8) and the belly section (4) and/or the back section (6) is discontinuous along the leg openings (19).

19. Method according to any one or more of the preceding claims, **characterized in that** the joining of the superposed partial webs (122, 124) for forming side seam areas (14) of the incontinence articles to be produced, and the performing of the separating cut is done in the same method step.

## Revendications

1. Procédé continu pour la fabrication d'articles pour personnes incontinentes (2) se présentant sous la forme d'une culotte, destinés à recevoir les excrétions corporelles, comprenant une section ventrale avant (4) et une section dorsale arrière (6), lesquelles sont reliées entre elles en usine au niveau des deux côtés des zones de couture latérales (14) pour former une bande ventrale et dorsale d'un seul tenant dans le sens transversal ou dans le sens de la circonférence des hanches (16) avec une ouverture de hanche (18) fermée dans le sens de la circonférence des hanches (16), et comprenant une section d'entrejambe (8) présentant un corps absorbant (7), laquelle section d'entrejambe s'étend dans le sens longitudinal (9) entre la section ventrale (4) et la section dorsale (6) et est jointe de façon inamovible à la section ventrale (4) et à la section dorsale (6), dans lesquels à la fois la section d'entrejambe (8) et la section ventrale (4) et la section dorsale (6) définissent des ouvertures de jambes (19) de l'article pour personnes incontinentes, la section ventrale (4) et la section dorsale (6) présentant à cet effet des premiers moyens d'élastification (28) qui s'étendent à distance les uns des autres et parallèlement entre eux en direction transversale ou dans le sens de la circonférence de hanche (16) et élastifient ainsi de façon plane la section ventrale (4) et la section dorsale (6), des deuxièmes moyens d'élastification (40, 42) étant prévus dans une zone (22, 26) de la section ventrale (4) et de la section dorsale (6) orientée vers l'entrejambe et les ouvertures de jambes (19), lesquels deuxièmes moyens d'élastification s'étendent depuis les deux zones de couture latérales (14) en direction (56) d'un axe médian longitudinal (44) de l'article pour personnes incontinentes (2) et s'étendent de façon incurvée de plus en plus distancés les uns des autres, le procédé comprenant les étapes consistant à :

   - amener des sections d'entrejambe (8) comportant des corps absorbants (7) et présentant des découpes de jambes (116) obtenues par une première coupe de contour (112),
   - amener sur la base de non-tissé deux bandes partielles (122, 124) qui formeront la future section ventrale (4) et la future section dorsale (6) de l'article pour personnes incontinentes (2),
   - amener et appliquer les deuxièmes moyens d'élastification (40, 42) sur les deux bandes partielles (122, 124) et les fixer à ces dernières,
   - assembler les sections d'entrejambe (8) avec les deux bandes partielles (122, 124) de façon à ce que les sections d'entrejambe (8) dans le sens longitudinal (9) transversalement au sens de la machine (120) viennent chevaucher à une extrémité une bande partielle (122) et à l'autre extrémité l'autre bande partielle (124), et à ce que les sections d'entrejambe (8) soient agencées à distance les unes des autres dans le sens de la machine (120), puis à fixer les sections d'entrejambe (8) et les bandes partielles (122, 124) dans les zones de chevauchement et les faire avancer dans le sens de la machine (120),
   - amener, appliquer et fixer les premiers moyens d'élastification (28) dans le sens de la machine (120) sur les bandes partielles (122, 124),
   - réaliser une deuxième coupe de contour (142) dans les bandes partielles (122, 124) au niveau de leurs sections de bord (58, 60) afin de former des découpes de jambes essentiellement incurvées (146),
   - plier autour d'une ligne de pliage (150) s'étendant dans le sens de la machine (120) de façon à ce que la bande partielle (122) vienne reposer sur l'autre bande partielle (124),
   - assembler les bandes partielles (122,124) reposant l'une sur l'autre transversalement au sens de la machine (120) à distance l'une de l'autre afin de former des zones de couture latérales (14) des articles pour personnes incontinentes à fabriquer (2) et obtenir ainsi des produits présentant une section ventrale (4), une section dorsale (6) et une section d'entrejambe (8) agencée entre les deux,
   - réaliser une coupe de séparation transversalement au sens de la machine (120) et obtenir ainsi plusieurs articles pour personnes incontinentes (2) finis.

2. Procédé selon la revendication 1, **caractérisé en ce que**, après l'étape consistant à amener, appliquer et fixer les deuxièmes moyens d'élastification (40, 42), on amène et on applique une couche de revêtement en non-tissé (126, 128) venant recouvrir les deuxièmes moyens d'élastification (40, 42) sur chaque bande partielle (122, 124) et on achemine les deux bandes partielles (122, 124) dans le sens de la machine à distance l'une de l'autres et parallèlement l'une à l'autre.

3. Procédé selon la revendication 1 ou 2, **caractérisé en ce que**, après l'étape consistant à amener, appliquer et fixer les premiers moyens d'élastification (28), on amène et on applique une couche de revêtement en non-tissé (134, 136) venant recouvrir les premiers moyens d'élastification sur chaque bande partielle (122, 124) et **en ce qu'**on achemine les deux bandes partielles (122, 124) dans le sens de la machine à distance l'une de l'autre et parallèlement l'une à l'autre.

**4.** Procédé selon la revendication 1, 2 ou 3, **caractérisé en ce que** l'on effectue la première coupe de contour (112) pour former les découpes de jambes (116) au niveau des sections d'entrejambe (8) au sein même du procédé continu.

**5.** Procédé selon la revendication 4, **caractérisé en ce que** les sections d'entrejambe (8) sont réalisées au sein du procédé continu, une bande de matériau de feuille supérieure sans fin (64), une bande de matériau de feuille arrière sans fin (62) et des corps absorbants (7) étant pour ce faire amenés dans le sens de la machine, les corps absorbants (7) étant agencés à distance les uns des autres entre la bande de matériau de feuille supérieure (64) et la bande de matériau de feuille arrière (62), **en ce que** l'assemblage ainsi obtenu est fixé sur lui-même et **en ce que** dans cet assemblage, on effectue la première coupe de contour (112) pour former les découpes de jambes (116) au niveau des sections d'entrejambe (8).

**6.** Procédé selon une ou plusieurs des revendications précédentes, **caractérisé en ce que** l'on amène aux sections d'entrejambe (8) des moyens d'élastification des jambes (82), en particulier entre la bande de matériau de feuille supérieure (64) et la bande de matériau de feuille arrière (62).

**7.** Procédé selon la revendication 6, **caractérisé en ce que** les moyens d'élastification des jambes (82) sont amenés de façon à s'étendre à des distances variables du corps absorbant (7) et à présenter, au niveau de leurs extrémités longitudinales, une distance plus grande par rapport au corps absorbant (7) qu'en leur centre.

**8.** Procédé selon la revendication 5, 6 ou 7, **caractérisé en ce que** la bande de matériau de feuille arrière (62) est amenée sous la forme d'une bande de feuille imperméable aux liquides pourvue d'un revêtement de non-tissé et/ou d'un revêtement de renfort (94).

**9.** Procédé selon une ou plusieurs des revendications précédentes, **caractérisé en ce que** l'on amène d'abord une bande de non-tissé sans fin qui est ensuite coupée le long du sens de la machine pour former les deux bandes partielles (122, 124).

**10.** Procédé selon une ou plusieurs des revendications précédentes, **caractérisé en ce que** les premiers moyens d'élastification (28) s'étendant dans le sens de la circonférence de hanche (16) sont appliqués avec une distance comprise entre 4 et 10 mm les uns des autres, en particulier à une distance comprise entre 4 et 8 mm, en particulier à une distance comprise entre 4 et 6 mm.

**11.** Procédé selon une ou plusieurs des revendications précédentes, **caractérisé en ce que** les deuxièmes moyens d'élastification (40, 42) sont appliqués à une distance variable les uns des autres, à une distance allant de 3 à 35 mm.

**12.** Procédé selon une ou plusieurs des revendications précédentes, **caractérisé en ce que** les premiers et/ou les deuxièmes moyens d'élastification (28, 40, 42) sont fixés aux bandes partielles sous une précontrainte allant de 1,5 à 6,0, en particulier de 2,5 à 5,0.

**13.** Procédé selon une ou plusieurs des revendications précédentes, **caractérisé en ce que** les deuxièmes moyens d'élastification (40, 42) sont désactivés dans une zone de chevauchement avec le corps absorbant (7).

**14.** Procédé selon une ou plusieurs des revendications précédentes, **caractérisé en ce que** la deuxième coupe de contour (142) est réalisée le long de et à une distance (D) du deuxième moyen d'élastification (40, 42) situé le plus près de la section d'entrejambe dans le sens longitudinal.

**15.** Procédé selon la revendication 14, **caractérisé en ce que** la distance (D) est comprise entre 2 et 40 mm, en particulier entre 3 et 30 mm et plus particulièrement entre 4 et 20 mm.

**16.** Procédé selon une ou plusieurs des revendications précédentes, **caractérisé en ce que** la deuxième coupe de contour (142) englobe uniquement les bandes partielles (122, 124), mais pas la section d'entrejambe (8).

**17.** Procédé selon une ou plusieurs des revendications précédentes, **caractérisé en ce que** la découpe de jambes (146) incurvée de la bande partielle (122, 124) qui formera la future section ventrale (4) présente un autre tracé, en particulier un autre angle ou rayon que la découpe de jambes (146) incurvée de la bande partielle (122, 124) qui formera la future section dorsale (6).

**18.** Procédé selon une ou plusieurs des revendications précédentes, **caractérisé en ce qu'**il est prévu le long des ouvertures de jambes (19) un tracé intermittent allant de la section d'entrejambe (8) jusqu'à la section ventrale (4) et/ou la section dorsale (6).

**19.** Procédé selon une ou plusieurs des revendications précédentes, **caractérisé en ce que** l'assemblage des bandes partielles (122, 124) venant l'une au-dessus de l'autre en vue de former les zones de couture latérales (14) des articles pour personnes incontinentes et la réalisation de la coupe de sépara-

**EP 2 180 865 B1**

tion sont réalisés au cours d'une seule et même étape.

*Fig. 1*

Fig. 2

Fig. 3

Fig. 4

Fig. 5

Fig. 6

Fig. 7

EP 2 180 865 B1

*Fig. 8*

*Fig. 9*

22

Fig. 10

EP 2 180 865 B1

Fig. 11

EP 2 180 865 B1

*Fig. 12*

EP 2 180 865 B1

Fig. 13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102007055524

- WO 2004052260 A1